# EUROPÄISCHE PATENTANMELDUNG

(11) **EP 4 850 728 A2**
(43) Veröffentlichungstag der Anmeldung: **09.12.2026**
(21) Anmeldenummer: 26212694.9
(22) Anmeldetag: 20.07.2020
(51) Int. Cl.: A61M 16/00

(54) **ATEMZUGANALYSATOR, BEATMUNGSGERÄT UND VERFAHREN ZUR ATEMZUGANALYSE**

(30) Priorität: 26.07.2019 DE 102019005280
(62) Dokumentnummer(n) der früheren Anmeldung(en) nach Art. 76 EPÜ: 20000261.6 / 3 769 668
(71) Anmelder: Löwenstein Medical Technology S.A., 2557 Luxembourg (LU)
(72) Erfinder: Schwaibold, Matthias, 76228 Karlsruhe (DE); Dömer, Benno, 76275 Ettlingen (DE); Verhoeven, Jan, 76275 Ettlingen (DE)
(74) Vertreter: Löwenstein Medical IP

(57) **Zusammenfassung**

Die Erfindung betrifft einen Atemzuganalysator (1) zur Erkennung von Atmungsereignissen einer mit einem Atemgas beatmeten Person, aufweisend eine elektronische Rechen- und Speichereinheit (2), die ausgebildet und eingerichtet ist, ein einem Beatmungsdruck und/oder einem Atemfluss und/oder einem Atemvolumen des der Person zugeführten Atemgases entsprechendes Signal (3) zu empfangen und während einer vorbestimmten Analysezeitdauer mittels eines Verlaufsanalysators (4) einen Verlauf des Signals (3) zu erfassen. Die Rechen- und Speichereinheit (2) ist ausgebildet und eingerichtet, aus dem Signalverlauf einen Signalausschnitt (5) zu bestimmen, der anhand einer vordefinierten oder einer aus dem Signalverlauf erkannten Ausschnittzeitdauer (6) kürzer als die Analysezeitdauer festgelegt ist, und den bestimmten Signalausschnitt (5) mittels eines Komparators (7) einer von mehreren Atemzugklassen zuzuordnen und jeweils eine Auftrittshäufigkeit der während der Analysezeitdauer zugeordneten Atemzugklasse in einem für jede Atemzugklasse vorgesehenen Häufigkeitszähler (8) in der Speichereinheit (2) zu speichern sowie wenigstens einen, jedoch weniger als die im Häufigkeitszähler (8) erfasste Auftrittshäufigkeit, der während der Analysezeitdauer bestimmten Signalausschnitte (5) für jede zugeordnete Atemzugklasse in der Speichereinheit (2, 9) zu speichern.

Die Erfindung betrifft ferner ein Beatmungsgerät zur Beatmung einer Person mit einem Atemgas, das einen erfindungsgemäßen Atemzuganalysator aufweist, sowie ein Verfahren zur Erkennung von Atmungsereignissen einer mit einem Atemgas beatmeten Person.

## Beschreibung

Die vorliegende Erfindung betrifft einen Atemzuganalysator zur Erkennung von Atmungsereignissen einer beispielsweise durch ein Beatmungsgerät mit einem Atemgas beatmeten Person nach dem Oberbegriff des Anspruchs 1. Die Erfindung betrifft ferner ein Beatmungsgerät zur Beatmung einer Person mit einem Atemgas, sowie ein Verfahren zur Erkennung von Atmungsereignissen einer mit einem Atemgas beatmeten Person.

Bei Beatmungsgeräten zur Beatmung von Personen mit einem Atemgas ist es oft von Vorteil, wenn die Möglichkeit einer Erkennung von Atemstörungen während der Beatmung besteht. Eine solche Erkennung ermöglicht einerseits eine Kontrolle der Effizienz der Beatmung durch das Beatmungsgerät und andererseits können Grund- und Nebenerkrankungen besser erkannt werden. Zudem kann die Steuerung des Beatmungsgerätes entsprechend der erkannten Atemmuster oder Ereignisse (dynamisch) und individuell an die zu beatmende Person angepasst werden.

Ein Beatmungsgerät mit einer Vorrichtung zur Überwachung von Beatmungsparametern ist beispielsweise aus der EP 1 136 094 B1 bekannt. Mit einem Sensor wird ein zeitlicher Verlauf des zu überwachenden Beatmungsparameters erfasst und einem Analysator bereitgestellt, der eine Mustererkennung durchführt und mit einem Klassifizierer zur Auswertungsunterstützung sowie mit einem Speicher gekoppelt ist, der für die Mustererkennung benötigte Vergleichsmuster bereitstellt.

Vor diesem Hintergrund liegt der vorliegenden Erfindung die Aufgabe zugrunde, einen Atemzuganalysator zur Erkennung von Atmungsereignissen einer mit einem Atemgas beatmeten Person, ein Beatmungsgerät zur Beatmung einer Person mit einem Atemgas sowie ein Verfahren zur Erkennung von Atmungsereignissen einer mit einem Atemgas beatmeten Person bereitzustellen, die einen für die Auswertung der während einer gesamten Analysezeitdauer erkannten Atmungsereignisse erforderlichen Zeitaufwand signifikant reduziert. Zudem sollen der Atemzuganalysator, das Beatmungsgerät sowie das Atemzuganalyseverfahren kompakt und mit technischen Mitteln verhältnismäßig geringer Komplexität und somit kostengünstig implementierbar sein.

Diese Aufgabe wird durch einen Atemzuganalysator mit den Merkmalen des Anspruchs 1, durch ein Beatmungsgerät mit den Merkmalen des Anspruchs 15 sowie durch ein Verfahren zur Atemzuganalyse mit den Merkmalen des Anspruchs 19 gelöst. Weitere, besonders vorteilhafte Ausgestaltungen der Erfindung offenbaren die jeweiligen Unteransprüche.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Erfindungsgemäß weist ein Atemzuganalysator zur Erkennung von Atmungsereignisses einer mit einem Atemgas beatmeten Person eine elektronische Rechen- und Speichereinheit auf, die beispielsweise in Form einer prozessorbasierten elektronischen Steuereinrichtung (z. B. Mikroprozessor, Mikrocontroller, DSP etc.) und wenigstens eines mit der Steuereinrichtung zusammenwirkenden elektronischen Speichers (z. B. RAM, ROM, EPROM, Flash, Magnetspeicher etc.) realisiert sein kann. Die Rechen- und Speichereinheit ist erfindungsgemäße ausgebildet und eingerichtet, ein einem Beatmungsdruck und/oder einem Atemfluss und/oder einem Atemvolumen des der Person zugeführten Atemgases entsprechendes Signal, insbesondere elektronisches Signal, zu empfangen und während einer vorbestimmten Analysezeitdauer mittels eines Verlaufsanalysators einen Verlauf des Signals (hierin nachstehend auch als Signalverlauf bezeichnet) zu erfassen.

Weiterhin ist die Rechen- und Speichereinheit gemäß der Erfindung ausgebildet und eingerichtet, aus dem ermittelten Signalverlauf (insbesondere unmittelbar nach seiner Ermittlung) einen Signalausschnitt zu bestimmen, der anhand einer vordefinierten oder einer aus dem Signalverlauf erkannten Ausschnittzeitdauer festgelegt ist, wobei die Ausschnittzeitdauer erheblich kürzer ist als die (gesamte) Analysezeitdauer. Die Analysezeitdauer kann beispielsweise im Bereich von mehreren Minuten bis zu einer oder mehreren Stunden oder sogar bis zu einem oder mehreren Tagen betragen, wohingegen die Ausschnittdauer bevorzugt regelmäßig eine Zeitdauer im Bereich von einer oder einigen wenigen Sekunden bis eine Minute, insbesondere bevorzugt bis maximal eine, zwei oder höchstens drei Minuten, umfasst.

Die Rechen- und Speichereinheit ist weiter ausgebildet und eingerichtet, den bestimmten Signalausschnitt mittels eines Komparators einer von mehreren Atemzugklassen zuzuordnen (hierin auch als zugeordnetes/erkanntes Atemmuster oder auch kurz als Atemmuster bezeichnet) und jeweils eine Auftrittshäufigkeit der während der Analysezeitdauer bzw. des gesamten Analysezeitraums zugeordneten (d. h. aufgetretenen) Atemzugklasse in einem für jede Atemzugklasse vorgesehenen Häufigkeitszähler in der Speichereinheit zu speichern sowie wenigstens einen, jedoch stets weniger als die im Häufigkeitszähler erfasste Auftrittshäufigkeit (d. h. die Gesamtzahl erkannter Signalausschnitte einer bestimmten Atemzugklasse während des gesamten Analysezeitraums), der während der Analysezeitdauer bestimmten Signalausschnitte für jede zugeordnete (d. h. aufgetretene) Atemzugklasse in der Speichereinheit zu speichern. Die Zuordnung des Signalausschnitts zu einer Atemzugklasse bedeutet, dass ein oder mehrere bestimmte Merkmale des Signalverlaufs im Signalausschnitt mit Merkmalen einer der mehreren Atemzugklassen übereinstimmen oder zumindest ähnlich sind.

Anhand des Häufigkeitszählers lässt sich selbst bei verhältnismäßig langen Analysezeiträumen, zum Beispiel mehrere Stunden bis zu mehreren Tagen, bereits mit sehr geringem Zeitaufwand ein Befund aus den nur wenigen, jedoch relevanten gespeicherten Analyseergebnissen für die beatmete Person erstellen. Zudem erfordern die Häufigkeitszähler (selbst bei einer großen Anzahl unterschiedlicher Atemzugklassen) einen sehr geringen Bedarf an zur Verfügung zu stellenden Speicherressourcen. Selbst wenn je Atemzugklasse ein oder einige wenige gespeicherte Signalausschnitte hinzugerechnet werden, so beträgt der Speicherbedarf des erfindungsgemäßen Atemzuganalysators insgesamt dennoch lediglich einen Bruchteil dessen, was eine kontinuierliche Speicherung des ursprünglich empfangenen Verlaufs des den Beatmungsdruck und/oder den Atemfluss und/oder das Atemvolumen des der beatmeten Person zugeführten Atemgases repräsentierenden Signals erfordern würde. Zudem werden gemäß der Erfindung stets weniger als alle im Analysezeitraum erkannten Signalausschnitte ein und derselben Atemzugklasse gespeichert, so dass sich die Befundung anhand der gespeicherten Signalausschnitte auf lediglich einige (wenige) relevante, exemplarische Signalausschnitte beschränkt, was den Zeitaufwand für die Befundung, insbesondere hinsichtlich des Vorliegens gesundheitskritischer Atmungszustände der beatmeten Person, erheblich senkt. Ist zur Befundung eine (drahtgebundene oder drahtlose) Datenübertragung aus der Speichereinheit zu einer beispielsweise entfernt vom Atemzuganalysator angeordneten Auswerteeinheit bzw. einem Expertenanwender (z. B. Arzt) vorgesehen bzw. erforderlich, reduziert sich in vorteilhafter Weise ebenfalls der Zeitaufwand für eine derartige Datenübertragung signifikant. Zudem kann die Speichereinheit selbst für verhältnismäßig langandauernde Überwachungsszenarien und Atmungsanalysen mit einem kleinen Speicher ausgestattet sein, was sowohl Bauraum zur Implementierung des Atemzuganalysators als auch Kosten für dessen Herstellung spart.

Es ist zu verstehen, dass der Verlaufsanalysator und/oder der Komparator nicht zwingend gegenständliche Funktionseinheiten sein müssen, sondern zum Beispiel als softwarebasierte Funktionseinheiten realisiert sein können, die von der Recheneinheit in entsprechender Weise ausgeführt werden, um ihre hierin beschriebene Funktion bereitzustellen.

Gemäß einer vorteilhaften Ausgestaltung der Erfindung ist die Rechen- und Speichereinheit ausgebildet und eingerichtet, je Atemzugklasse lediglich eine vorbestimmte Maximalanzahl an Signalausschnitten in der Speichereinheit zu speichern. Mit anderen Worten kann zu Beginn eines Analysezeitraums oder einmalig vor der ersten Inbetriebnahme des Atemzuganalysators eine maximale Anzahl an zu speichernden Signalausschnitten je Atemzugklasse vorgegeben werden, zum Beispiel maximal 3, 5 oder 10 Signalausschnitte. Bevorzugt sind maximal fünf Signalausschnitte pro Atemzugklasse von dem Atemzuganalysator zu speichern, besonders bevorzugt maximal drei. So liegen einerseits für eine Befundung ausreichend viele Beispiele von je Atemzugklasse erkannten, relevanten Signalverläufen vor und andererseits ist der Speicherbedarf für eine derart geringe Maximalzahl zu speichernder Signalausschnitte stets sehr gering.

Je nach Anwendungsfall kann es vorgesehen sein, dass die maximale Anzahl zu speichernder Signalausschnitte je Atemzugklasse die ersten während einer gesamten Analysezeitdauer erkannten, zugeordneten Atemmuster sind oder zum Beispiel die zuletzt erkannten (aktuellsten) Atemmuster während der Analysezeitdauer.

Für eine Priorisierung von Atemzugklassen, beispielsweise bezüglich des Schweregrads des mit der jeweiligen Atemzugklasse verbundenen gesundheitlichen Komplikationszustands für den Patienten, sieht eine vorteilhafte Ausgestaltung der Erfindung vor, dass die Anzahl der mehreren zu speichernden Signalausschnitte für wenigstens zwei unterschiedliche Atemzugklassen separat festlegbar ist. So kann für eine gesundheitlich besonders relevante Atemzugklasse beispielsweise eine höhere Maximalzahl an zu speichernden, während der Analysezeitdauer erkannten, zugeordneten Atemmustern vorgegeben werden, wohingegen für weniger kritische Atemzugklassen eine geringere Anzahl zu speichernder Signalausschnitte bestimmt sein kann. So ist eine optimale, effiziente Speicherausnutzung erzielbar bei weiterhin geringem Speicherbedarf zur Speicherung aller Signalausschnitte, obwohl für eine genaue Befundung bei bestimmten Atemzugklassen eine höhere Anzahl gespeicherter Atemmuster im Vergleich zu anderen Atemzugklassen erforderlich sein kann.

Eine noch weitere vorteilhafte Ausgestaltung der Erfindung sieht vor, dass die Ausschnittzeitdauer des Signalausschnitts anhand wenigstens eines vorbestimmten Signalverlaufskriteriums festlegbar ist. Dieses ist vorzugsweise aus einer Gruppe von Kriterien ausgewählt, die wenigstens eine Steigung und/oder einen Steigungsverlauf des erfassten Signalverlaufs, ein Auftreten wenigstens eines Maximums und/oder Minimums des Signalverlaufs und dergleichen beinhaltet. Eine solche dynamische, automatische Festlegung der Ausschnittzeitdauer ist gegenüber einer beispielsweise zu Beginn der Analysezeitdauer einmalig fest vorgegebenen Ausschnittzeitdauer erheblich flexibler und ermöglicht eine automatische Adaption an die augenblickliche Atmungssituation der zu beatmenden Person. So können die vorgenannten Kriterien beispielsweise genutzt werden, einen Anfang und ein Ende wenigstens eines vollständigen Atemzugs zu erkennen und diesen als zu analysierenden Signalausschnitt zu bestimmen. Ebenso kann auch nur ein bestimmter Signalverlaufsteil eines Atemzugs in dem bestimmten Signalausschnitt enthalten sein. Als Steigung kann die Änderung des Signalverlaufs zum Beispiel über der Zeit verstanden werden. Aus einem Steigungsverlauf lässt sich - zum Beispiel bei einem Vorzeichenwechsel (Nulldurchgang des Steigungsverlaufs) - auf einen Anstieg des Signals im Signalverlauf gefolgt von einem Abfall des Signals und umgekehrt schließen. Somit ist anhand des Steigungsverlaufs auch eine Konturbestimmung des eigentlichen Signalverlaufs möglich.

Besonders bevorzugt kann die Ausschnittzeitdauer derart bemessen sein, dass der Signalausschnitt wenigstens einen vollständigen Atemzug beinhaltet, vorzugsweise 1 bis maximal 10 Atemzüge, besonders bevorzugt wenigstens 3 bis maximal 5 Atemzüge.

Eine weitere vorteilhafte Ausgestaltung der Erfindung sieht vor, dass der gespeicherte Signalausschnitt mit einem Zeitstempel, zum Beispiel Uhrzeit und/oder Datum, seiner Bestimmung versehen ist, so dass sich das Auftreten des jeweils erkannten Atemmusters bei einer späteren Befundung in einfacher Weise feststellen und bei der Auswertung entsprechend berücksichtigen lässt.

Des Weiteren sieht eine andere vorteilhafte Ausgestaltung der Erfindung vor, dass der Komparator ausgebildet und eingerichtet ist, den Signalausschnitt der jeweiligen Atemzugklasse mittels Mustererkennung und/oder mittels eines Vergleichs des Signalverlaufs im Signalausschnitt mit einer vorbestimmten Anzahl an vorab in der Speichereinheit gespeicherten, vordefinierten Referenzsignalverläufen und/oder mittels einer erfassten Steigung und/oder eines Steigungsverlaufs des Signalverlaufs im Signalausschnitt und/oder mittels eines oder mehrerer erfasster Maxima und/oder Minima des Signalverlaufs im Signalausschnitt zuzuordnen. Die Steigung bzw. der Steigungsverlauf ermöglichen die Ermittlung einer bestimmten Kontur des Signalverlaufs im Signalausschnitt.

Gemäß einer noch weiteren vorteilhaften Ausgestaltung der Erfindung ist der Komparator ausgebildet und eingerichtet, eine vorbestimmte Anzahl an ausgewählten, den jeweiligen Atemzugklassen während der Analysezeitdauer zugeordneten (d. h. erkannten bzw. aufgetretenen) Signalausschnitten als zusätzliche, neu hinzugefügte, adaptiv gelernte Referenzsignalverläufe in der Speichereinheit zu speichern und bei der Zuordnung nachfolgend erfasster Signalausschnitte zu den Atemzugklassen zu berücksichtigen. Somit lässt sich während der Analysezeitdauer eine automatische, dynamische Anpassung des Atemzuganalysators an eine personenindividuelle Atmung erzielen, um die Qualität des Analyseergebnisses (Klassifizierungsrate und -konfidenz) weiter zu steigern.

Nach einer bevorzugten weiteren Ausgestaltung der Erfindung beinhalten die Atemzugklassen wenigstens die Atemzugstypen Inspiration, Exspiration, Pause und Husten. Vorzugsweise beinhalten die Atemzugklassen zusätzlich auch Atemzugtypen, die eine inspiratorische Flusslimitation und/oder exspiratorische Flusslimitation und/oder einen intrinsischen PEEP ("positive end-expiratory pressure") und/oder Atemzug-Asynchronietypen wie eine ineffiziente Atemzugsbemühung (z. B. ineffiziente Inspirationsbemühung, fehlender Inspirationstrigger) und/oder eine doppelte Atemzugbemühung (z. B. weitere Inspirationsbemühung zu Beginn einer Exspiration infolge einer zu kurzen Inspirationszeit, die hierin auch als Doppeltrigger bezeichnet wird) repräsentieren. Die Asynchronietypen repräsentieren insbesondere eine Asynchronie zwischen der Atmung der beatmeten Person und dem (beispielsweise durch ein Beatmungsgerät vorgegebenen) Beatmungsdruck des Atemgases. Die Erkennung von Ereignissen wie inspiratorische Flusslimitation, fehlender Inspirationstrigger, Hustenstoß, exspiratorische Flusslimitation, intrinsischer PEEP und dergleichen kann vom Atemzuganalysator beispielsweise mittels eines Vergleichs des Signalverlaufs im Signalausschnitt mit einer vorbestimmten Anzahl an vorab in der Speichereinheit gespeicherten, vordefinierten und/oder adaptiv gelernten Referenzsignalverläufen und/oder mittels einer erfassten Steigung und/oder eines Steigungsverlaufs des Signalverlaufs im Signalausschnitt und/oder mittels eines oder mehreren erfassten Maxima und/oder Minima des Signalverlaufs im Signalausschnitt erfolgen.

Nach einer noch weiteren vorteilhaften Ausgestaltung ist der Komparator ausgebildet und eingerichtet, Zusatzinformationen über den Atemzugtyp wie spontaner oder mandatorischer Atemzug und/oder über eine Drehzahl eines der Person das Atemgas zuführenden Beatmungsgebläses und/oder über einen Leckageverlust des der Person zugeführten Atemgases zu empfangen und bei der Zuordnung des Signalausschnitts zu den Atemzugklassen zu berücksichtigen. Die Zusatzinformationen können beispielsweise verwendet werden, den Signalverlauf im Signalausschnitt für eine genauere Zuordnung zur richtigen Atemzugklasse durch den Komparator vorab einer entsprechenden Vorverarbeitung (z. B. Normalisierung des Signalverlaufs) zu unterziehen.

Erfindungsgemäß können interne und/oder optionale externe oder adaptierbare Sensoren genutzt werden, um die Informationen über die Atemtätigkeit oder Atemgaswerte oder - Parameter zu liefern. Insbesondere für die Identifikation von Atembemühungen oder der Atemtätigkeit können Beschleunigungssensor auf dem Oberkörper, Dehnungs- oder Spannungssensor in einem Gurt um den Oberkörper, EMG-Sensoren an den Atemmuskeln oder dem Zwerchfell, ein Drucksensor in den Atemwegen, Impedanzsensoren in einem Gurt um den Oberkörper, Messung der Thoraximpedanz über Elektroden, Messung von Atemgeräuschen, Messung von SpO2 oder transkutanem CO2 oder CO2 in der Ausatemluft, Messung von Puls oder EKG jeweils alleine oder kumulativ verwendet werden. Die Signalverläufe dieser Sensoren können entsprechend der Prinzipien dieser Erfindung ausgewertet werden und können alleine in Kombination verwendet werden die Vorgaben von Druck und/oder Fluss und Oder Volumen des Beatmungsgerätes so zu verbessern, dass weniger oder weniger schwerwiegende Atemereignisse auftreten.

Es ist anzumerken, dass die Auswertung des Signalverlaufs im Signalausschnitt anhand eines zeitlichen Verlaufs des Signals, zum Beispiel des Atemflusses über der Zeit, erfolgen kann. Alternativ oder zusätzlich kann auch die Auswertung des Signals, zum Beispiel des Atemflusses oder Atemvolumens, über dem Beatmungsdruck (so genanntes P/V-Diagramm) erfolgen.

Als weiterer Aspekt der Erfindung wird ein Beatmungsgerät zur Beatmung einer Person mit einem Atemgas bereitgestellt, das eine Sensoreinheit zur Bestimmung eines Beatmungsdrucks und/oder eines Atemflusses und/oder eines Atemvolumens des der Person zugeführten Atemgases aufweist. Weiterhin weist das Beatmungsgerät gemäß der Erfindung einen Atemzuganalysator nach einer der vorstehenden Ausgestaltungen auf, wobei die Sensoreinheit mit der Rechen- und Speichereinheit des Atemzuganalysators in datenübertragender Weise gekoppelt ist und eingerichtet ist, ein dem bestimmten Beatmungsdruck und/oder dem bestimmten Atemfluss und/oder dem bestimmten Atemvolumen des Atemgases entsprechendes Signal zu erzeugen und der Rechen- und Speichereinheit zuzuführen.

Bezüglich beatmungsgerätbezogener Begriffsdefinitionen sowie der Wirkungen und Vorteile beatmungsgerätgemäßer Merkmale wird vollumfänglich auf die vorstehenden Erläuterungen sinngemäßer Definitionen, Wirkungen und Vorteile bezüglich des erfindungsgemäßen Atemzuganalysators verwiesen. Offenbarungen hierin bezüglich des erfindungsgemäßen Atemzuganalysators sollen in sinngemäßer Weise auch zur Definition des erfindungsgemäßen Beatmungsgeräts herangezogen werden können, sofern dies hierin nicht ausdrücklich ausgeschlossen ist. Ebenso sollen Offenbarungen hierin bezüglich des erfindungsgemäßen Beatmungsgeräts in sinngemäßer Weise zur Definition des erfindungsgemäßen Atemzuganalysators herangezogen werden können, sofern dies hierin nicht ebenfalls ausdrücklich ausgeschlossen ist. Insofern wird auf eine Wiederholung von Erläuterungen sinngemäß gleicher Merkmale, deren Wirkungen und Vorteile bezüglich des hierin offenbarten erfindungsgemäßen Atemzuganalysators sowie des hierin offenbarten erfindungsgemäßen Beatmungsgeräts zugunsten einer kompakteren Beschreibung weitgehend verzichtet.

Eine vorteilhafte Ausgestaltung der Erfindung sieht vor, dass das Beatmungsgerät wenigstens eine Datenübertragungsschnittstelle aufweist, die ausgebildet und eingerichtet ist, den in der Speichereinheit gespeicherten Inhalt des Häufigkeitszählers jeder Atemzugklasse und/oder den wenigstens einen in der Speichereinheit gespeicherten Signalausschnitt jeder zugeordneten (d. h. während der Analysedauer aufgetretenen) Atemzugklasse an einen Datenempfänger bzw. eine Datenempfangseinheit zu übertragen. Die Datenübertragung kann hierbei drahtgebunden oder drahtlos erfolgen. Datenübertragungsschnittstellen können beispielsweise ein elektronischer Datenübertragungsbus, eine Netzwerkschnittstelle (z. B. LAN) ein Datenübertragungsmodem, eine USB-Schnittstelle, eine Funkübertragungsschnittstelle wie z. B. Infrarot, Bluetooth, WIFI, GSM/LTE und dergleichen oder auch ein Wechselspeichermedium wie z. B. eine Speicherkarte (Flash), ein USB-Stick/-Festplatte und dergleichen sein. Der Datenempfänger bzw. die Datenempfangseinheit kann beispielsweise eine geräteexterne (entfernte) Datenverarbeitungseinrichtung mit einer Auswerteeinheit (z. B. Auswertesoftware) sein, ein Expertenanwender (z. B. Arzt), eine Anzeigeeinrichtung (z. B. Display, Monitor) und dergleichen. Die Datenempfangseinheit kann somit die Befundung des Analyseergebnisses zu einem späteren Zeitpunkt ermöglichen, insbesondere in dem Fall, in dem das Beatmungsgerät keine geräteinterne Auswerteeinheit aufweist. Das Beatmungsgerät kann jedoch alternativ oder zusätzlich eine interne Auswerteeinheit aufweisen.

Nach einer weiteren vorteilhaften Ausgestaltung der Erfindung weist das Beatmungsgerät eine weitere Sensoreinheit auf, die ausgebildet ist, eine Drehzahl eines Beatmungsgebläses und/oder einen Leckageverlust des Atemgases bei der Beatmung der Person und/oder einen Atemzugtyp wie spontane Atmung oder mandatorische Atmung zu erfassen und dem Atemzuganalysator zuzuführen.

Gemäß einer anderen vorteilhaften Weiterbildung weist das Beatmungsgerät eine Auswerteeinheit auf, die ausgebildet und eingerichtet ist, das Analyseergebnis des Atemzuganalysators hinsichtlich gesundheitskritischer Komplikationszustände der beatmeten Person auszuwerten und in der Speichereinheit zu speichern und/oder auf einer Anzeigeeinrichtung anzuzeigen und/oder an eine geräteexterne Datenempfangseinheit zu übertragen. Die Datenübertragung kann hierzu insbesondere über die vorgenannte Datenübertragungsschnittstelle erfolgen. Als Datenempfangseinheit sind insbesondere eine Überwachungseinheit ein Monitor in einem Krankenhaus oder Pflegeheim und bei häuslicher Beatmung ein Telemonitoring-Server zu verstehen. Falls die Häufigkeit und/oder der Schweregrad einer festgestellten Komplikation einen festgelegten Grenzwert überschreitet, kann das Beatmungsgerät ebenfalls ausgebildet sein, einen Alarm auszugeben, beispielsweise auf der Anzeigeeinrichtung und/oder einen Alarm an die Datenempfangseinheit zu senden.

Nach einer weiteren vorteilhaften Ausgestaltung der Erfindung ist vorgesehen, dass die Atemzugklasse ineffiziente Atemzugbemühung dadurch erkannt wird, dass der Atemfluss während der Exspirationsphase analysiert wird und dass der Atemfluss während der Exspirationsphase zumindest zeitweise eine Vorzeichenumkehr aufweist.

Nach einer weiteren vorteilhaften Ausgestaltung der Erfindung ist vorgesehen, dass die Atemzugklasse ineffiziente Atemzugbemühung dadurch erkannt wird, dass analysiert wird, ob der Ausatemfluss innerhalb eines ersten Zeitfensters zu Exspirationsbeginn eine bestimmte Schwelle (z.B. in l/min) überschreitet und danach innerhalb eines zweiten Zeitfensters abnimmt, bis er ein gewisse Schwelle unterschreitet oder sogar kurzzeitig ein positiver, also inspiratorischer Atemfluss entsteht oder zumindest die Ableitung des Atemflusses auf eine Abnahme des exspiratorischen Atemflusses hindeutet, wobei dann überprüft wird, ob der Ausatemfluss innerhalb eines dritten Zeitfensters wieder zunimmt, also erneut ein stärkerer Ausatemfluss entsteht oder zumindest die Ableitung des Atemflusses auf eine erneute Zunahme des exspiratorischen Atemflusses hindeutet, noch bevor die nächste Inspiration erkannt wird.

Nach einer weiteren vorteilhaften Ausgestaltung der Erfindung ist vorgesehen, dass die Atemzugklasse ineffiziente Atemzugbemühung dadurch erkannt wird, dass die Ineffiziente Inspirationsbemühung durch eine Mustererkennung erkannt wird, die den Verlauf des Atemflusses vergleicht mit zuvor gespeicherten Testmustern, die Ineffiziente Inspirationsbemühungen repräsentieren.

Nach einer weiteren vorteilhaften Ausgestaltung der Erfindung ist vorgesehen, dass die Atemzugklasse ineffiziente Atemzugbemühung dadurch erkannt wird, dass die Atemzugklasse Doppeltrigger erkannt wird durch einen Vergleich der Ausatemzeit mit einer Vielzahl von vorausgehenden (typischen) Ausatemzeiten oder einer durchschnittlichen Ausatemzeit oder einer vorgegebenen Ausatemzeit.

Nach einer weiteren vorteilhaften Ausgestaltung der Erfindung ist vorgesehen, dass die Atemzugklasse ineffiziente Atemzugbemühung dadurch erkannt wird, dass die Atemzugklasse Doppeltrigger erkannt wird durch einen Vergleich des gemessenen Flusses, mit einem theoretischen Atemfluss, wobei der gemessene Fluss der nach Umschaltung auf exspiratorischen Beatmungsdruck gegenüber dem theoretischen Atemfluss erhöht bleibt.

Nach einer weiteren vorteilhaften Ausgestaltung der Erfindung ist vorgesehen, dass die Atemzugklasse des Intrinsischen PEEP anhand von hinterlegten beispielhaften Kurvenverläufen von Fluss oder Volumen, die für einen Intrinsischen PEEP repräsentativ sind, erfolgt, wobei aktuelle Kurvenverläufe dafür mit den hinterlegten Kurvenverläufen verglichen werden.

Nach einer weiteren vorteilhaften Ausgestaltung der Erfindung ist vorgesehen, dass die Atemzugklasse des Intrinsischen PEEP über eine Auswertung der zeitlichen Fluss- oder Volumenverteilung innerhalb der Exspiration erfolgt, wobei am Anfang der Exspiration ein hoher Ausatemfluss erkannt wird, der anschließend abnimmt und im weiteren Verlauf der Exspiration in einen niedrigen Ausatemfluss übergeht.

Nach einer weiteren vorteilhaften Ausgestaltung der Erfindung ist vorgesehen, dass die Atemzugklasse des Intrinsischen PEEP über eine Erkennung einer Inspirationsbemühung des Patienten und eine Auswertung des Ausatemflusses im Zeitbereich der Inspirationsbemühung erfolgt, wobei der verbleibende exspiratorische Atemfluss im Zeitbereich des Auslösens der nächsten Inspirationsphase, repräsentativ für das Ausmaß des intrinsischen PEEP ist.

Nach einer weiteren vorteilhaften Ausgestaltung der Erfindung ist vorgesehen, dass die Atemzugklasse Hustenstoß über eine Mustererkennung des Exspirationsverlaufes von Fluss oder Volumen anhand von hinterlegten Verläufen erfolgt, die repräsentativ für Hustenstöße sind, wobei aktuelle Verläufe dafür mit den hinterlegten Verläufen verglichen werden.

Nach einer weiteren vorteilhaften Ausgestaltung der Erfindung ist vorgesehen, dass die Atemzugklasse Hustenstoß über einen Vergleich des Verlaufes des aktuellen Ausatemflusses, des Peak-Ausatemflusses oder -volumens mit den typischen vorhergehenden Ausatemflüssen, Peak-Ausatemflüssen oder -volumina des Patienten zur Erkennung einer forcierten Ausatmung erfolgt, die auf einen Hustenstoß hindeutet, wobei ein Hustenstoß erkannt wird, wenn der aktuelle Atemzug über 70% oder 140% oder 200% der typischen Atemzüge erreicht.

Gemäß einem noch weiteren Aspekt der Erfindung wird ein Verfahren zur Analyse von Atmungsereignissen einer mit einem Atemgas beatmeten Person bereitgestellt, bei dem mittels einer Sensoreinheit ein einem Beatmungsdruck und/oder einem Atemfluss und/oder einem Atemvolumen des der Person zugeführten Atemgases entsprechendes Signal erzeugt wird und während einer vorbestimmten Analysezeitdauer ein zeitlicher Verlauf des Signals ermittelt wird. Aus dem ermittelten Signalverlauf wird anschließend (insbesondere unmittelbar nach seiner Ermittlung) ein Signalausschnitt bestimmt, der anhand einer vordefinierten oder einer aus dem Signalverlauf erkannten Ausschnittzeitdauer, die kürzer ist als die Analysezeitdauer, festgelegt wird. Der bestimmte Signalausschnitt wird anschließend einer von mehreren Atemzugklassen zugeordnet, wobei jeweils eine Auftrittshäufigkeit der während der Analysezeitdauer zugeordneten Atemzugklasse in einem für jede Atemzugklasse vorgesehenen Häufigkeitszähler gespeichert wird sowie wenigstens einer, jedoch weniger als die im Häufigkeitszähler erfasste Auftrittshäufigkeit, der während der Analysezeitdauer bestimmten Signalausschnitte für jede zugeordnete Atemzugklasse gespeichert wird.

Als ein weiterer Aspekt der Erfindung kann das Beatmungsgerät auch intelligent oder autoadaptiv reagieren, um das Auftreten zumindest einiger Atemereignisse zu minimieren. Das Beatmungsgerät ist eingerichtet und ausgebildet einzelne Atemereignisse zu identifizieren und den Beatmungsdruck und/oder den Fluss und/oder das Volumen des Atemgases, getrennt für die Inspiration und für die Exspiration entsprechend dem Atemereignis oder der Häufigkeit oder Bedeutung des Atemereignisses derart zu verändern, dass das Atemereignis oder die Häufigkeit oder Bedeutung des Atemereignisses abgeschwächt wird oder abnimmt. Beispielsweise kann die Triggersensitivität, für die Erkennung von Atembemühungen des Patienten, bei erkannten Asynchronien, nämlich dann, wenn Atembemühungen des Patienten ausbleiben oder zu einem unerwarteten Zeitpunkt oder einem Zeitpunkt außerhalb eines Erwartungsfensters erfolgen, angepasst werden. Der exspiratorische Druck kann erhöht oder vermindert werden, er kann insbesondere zu Beginn der Exspiration erhöht und dann vermindert werden oder zum Ende der Exspiration erhöht werden. Der Druckverlauf bei der Umschaltung von dem inspiratorischen auf den exspiratorischen Druck (Schienung der Atemwege in der Exspiration) kann zur Minimierung des intrinsischen PEEPs angepasst werden.

Als ein weiterer Aspekt der Erfindung kann das Beatmungsgerät auch bei Erkennung einer inspiratorischen Flusslimitation und/oder einer exspiratorischen Flusslimitation und/oder eines intrinsischen PEEP und/oder eine ineffiziente Atemzugbemühung und/oder einer doppelten Atemzugbemühung (Doppeltrigger) zumindest eine Meldung über die Datenübertragungsschnittstelle und/oder am Display generieren oder in Daten speichern und/oder zumindest eine Einstellung der Beatmung verändert wird beispielsweise ausgewählt aus IPAP und/oder EPAP und/oder Inspirationszeit und/oder Exspirationszeit und/oder Triggerempfindlichkeit.

Es sei erneut angemerkt, dass bezüglich verfahrensbezogener Begriffsdefinitionen sowie der Wirkungen und Vorteile verfahrensgemäßer Merkmale vollumfänglich auf die vorstehenden Erläuterungen sinngemäßer Definitionen, Wirkungen und Vorteile bezüglich der erfindungsgemäßen Vorrichtung(en) verwiesen wird. Dementsprechend können Offenbarungen hierin bezüglich der erfindungsgemäßen Vorrichtung(en) in sinngemäßer Weise auch zur Definition des erfindungsgemäßen Verfahrens herangezogen werden und Offenbarungen hierin bezüglich des erfindungsgemäßen Verfahrens können in sinngemäßer Weise zur Definition der erfindungsgemäßen Vorrichtung(en) herangezogen werden. Auf eine Wiederholung von Erläuterungen sinngemäß gleicher Merkmale, deren Wirkungen und Vorteile wird somit weitgehend verzichtet.

Weitere Merkmale und Vorteile der Erfindung ergeben sich aus der folgenden Beschreibung nicht einschränkend zu verstehender Ausführungsbeispiele der Erfindung, die im Folgenden unter Bezugnahme auf die Zeichnung näher erläutert wird. In dieser Zeichnung zeigen schematisch:
- Fig. 1: ein Funktionsdiagramm eines Ausführungsbeispiels eines Atemzuganalysators gemäß der Erfindung,
- Fig. 2: eine Übersichtsdarstellung von mittels des Atemzuganalysators aus Fig. 1 gewonnenen Analyseergebnissen für einen kurzen Zeitraum,
- Fig. 3: eine weitere Übersichtsdarstellung von mittels des Atemzuganalysators aus Fig. 1 gewonnenen Analyseergebnissen für einen langen Zeitraum,
- Fig. 4a, b, c: beispielhafte Signalverläufe eines einem Beatmungsdruck, einem Atemfluss und einem Atemvolumen entsprechenden Signals einer ersten Atemzugklasse,
- Fig. 5a, b: beispielhafte Signalverläufe eines einem Beatmungsdruck, einem Atemfluss und einem Atemvolumen entsprechenden Signals einer zweiten Atemzugklasse,
- Fig. 6a, b: beispielhafte dritte Signalverläufe eines einem Beatmungsdruck, einem Atemfluss und einem Atemvolumen entsprechenden Signals einer dritten Atemzugklasse,
- Fig. 7a, b: beispielhafte vierte Signalverläufe eines einem Beatmungsdruck, einem Atemfluss und einem Atemvolumen entsprechenden Signals einer vierten Atemzugklasse und
- Fig. 8: eine perspektivische Ansicht eines Ausführungsbeispiels eines Beatmungsgeräts gemäß der Erfindung.

In den unterschiedlichen Figuren sind hinsichtlich ihrer Funktion gleichwertige Teile stets mit denselben Bezugszeichen versehen, so dass diese in der Regel auch nur einmal beschrieben werden.

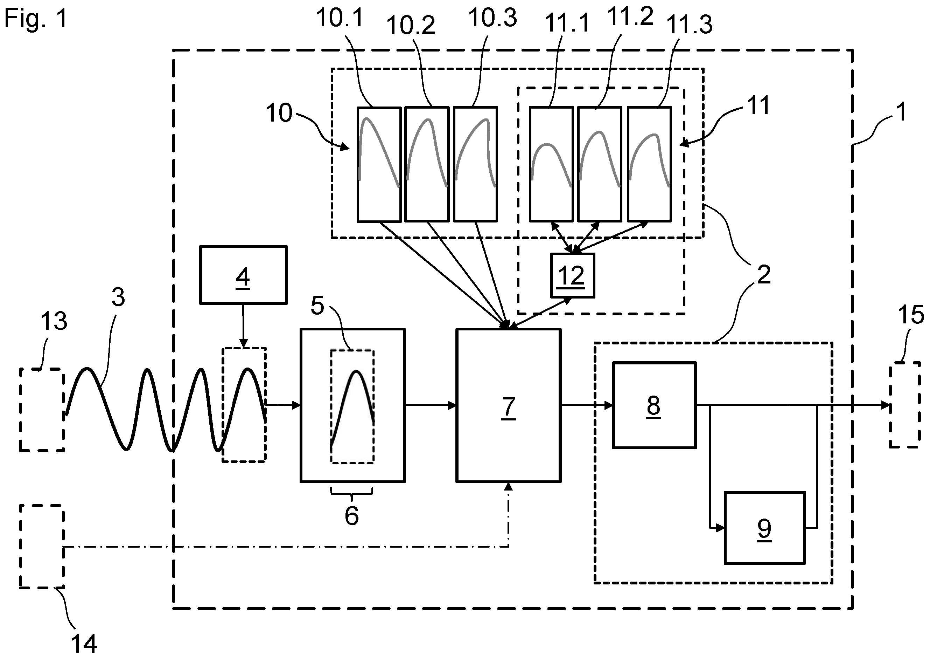

Fig. 1 stellt schematisch ein Funktionsdiagramm eines Ausführungsbeispiels eines Atemzuganalysators 1 zur Erkennung von Atmungsereignissen einer mit einem Atemgas beatmeten Person (nicht dargestellt) gemäß der Erfindung dar. Der dargestellte Atemzuganalysator 1 weist eine elektronische (vorliegend prozessorbasierte) Rechen- und Speichereinheit, von der lediglich die Speichereinheit 2 in Fig. 1 mit einer punktierten Umrandung schematisch dargestellt ist, auf, die ausgebildet und eingerichtet ist, ein einem Beatmungsdruck und/oder einem Atemfluss und/oder einem Atemvolumen des der Person zugeführten Atemgases entsprechendes Signal 3 zu empfangen und während einer vorbestimmten Analysezeitdauer mittels eines Verlaufsanalysators 4 einen Verlauf des Signals 3 zu ermitteln. Die Rechen- und Speichereinheit 2 des dargestellten Atemzuganalysators 1 ist weiter ausgebildet und eingerichtet, aus dem Signalverlauf einen Signalausschnitt 5 zu bestimmen, der vorliegend anhand einer aus dem Signalverlauf erkannten Ausschnittzeitdauer 6, die wesentlich kürzer ist als die Analysezeitdauer und beispielsweise einen oder auch einige wenige vollständige Atemzüge (z. B. 1-3 Atemzüge) umfassen kann, festgelegt ist, und den bestimmten Signalausschnitt 5 mittels eines Komparators 7 einer von mehreren vordefinierten Atemzugklassen zuzuordnen und jeweils eine Auftrittshäufigkeit der während der Analysezeitdauer zugeordneten Atemzugklasse in einem für jede Atemzugklasse vorgesehenen Häufigkeitszähler 8 in der Speichereinheit 2 zu speichern sowie wenigstens einen, jedoch weniger als die im Häufigkeitszähler 8 insgesamt erfasste Auftrittshäufigkeit, der während der Analysezeitdauer bestimmten Signalausschnitte 5 für jede zugeordnete Atemzugklasse in der Speichereinheit 2, in Fig. 1 in einem Speicherbereich 9 der Speichereinheit 2 für Signalausschnitte, zu speichern.

Fig. 1 ist weiter zu entnehmen, dass der Atemzuganalysator 1 mehrere vordefinierte Referenzsignalverläufe 10, von denen in Fig. 1 beispielhaft drei gezeigt sind, aufweist, die in der Speichereinheit 2 gespeichert sind. Es ist zu verstehen, dass je Atemzugklasse jeweils mehrere Referenzsignalverläufe 10.1 bis 10.n gespeichert sein können. Die vordefinierten Referenzsignalverläufe 10.1 bis 10.n können vom Komparator 7 für einen Vergleich des augenblicklich erfassten Signalausschnitts 5 herangezogen werden, um den Signalausschnitt 5 einer die Referenzsignalverläufe repräsentierenden Atemzugklasse zuzuordnen.

Bei dem in Fig. 1 gezeigten Atemzuganalysator 1 ist weiterhin angedeutet, dass optional (strichpunktierte Umrandung) während des Betriebs bzw. der Analysezeitdauer adaptiv gelernte weitere Referenzsignalverläufe 11 je Atemzugklasse, von denen in Fig. 1 beispielhaft drei adaptiv gelernte Referenzsignalverläufe 11.1, 11.2, 11.3 einer Atemzugklasse dargestellt sind, in der Speichereinheit 2 gespeichert sind. Es ist zu verstehen, dass die Anzahl sowohl an vordefinierten Referenzsignalverläufen 10 als auch an adaptiv gelernten Referenzsignalverläufen 11 je Atemzugklasse je nach Einsatzzweck des Atemzuganalysators 1 entsprechend vorgegeben sein kann. Für das adaptive Lernen der Referenzsignalverläufe 11 weist der Atemzuganalysator 1 optional eine adaptive Lerneinheit 12 auf, die zum Lernen und Erweitern der adaptiv gelernten Referenzsignalverläufe 11 einerseits Signalausschnittdaten von dem Komparator 7 empfängt und andererseits dem Komparator 7 bereits gelernte Referenzsignalverläufe 11 für die Zuordnung der Signalausschnitte 5 zu den jeweiligen Atemzugklassen zur Verfügung stellt.

In Fig. 1 ist weiterhin angedeutet, dass das Signal 3 von einer analysatorexternen Sensoreinheit 13 erzeugt wird. Ferner können dem Atemzuganalysator 1 über eine weitere, optionale zweite Sensoreinheit 14 wahlweise noch weitere Informationen zum Beispiel über den Atemzugtyp, insbesondere spontaner oder mandatorischer Atemzug, und/oder über eine Drehzahl eines der Person das Atemgas zuführenden Beatmungsgebläses (nicht dargestellt) und/oder über einen Leckageverlust des der Person zugeführten Atemgases zugeführt werden.

Außerdem können wenigstens die in der Speichereinheit 2 gespeicherten, während des Analysezeitraums gewonnenen Analysedaten, vorliegend insbesondere der Inhalt des jeweiligen Häufigkeitszählers 8 je Atemzugklasse und/oder der Inhalt des Speicherbereichs 9, der die je Atemzugklasse erkannten Signalausschnitte während der Analysezeitdauer speichert, über eine optionale Datenübertragungsschnittstelle 15 an einen in Fig. 1 nicht dargestellten Datenempfänger übertragen werden.

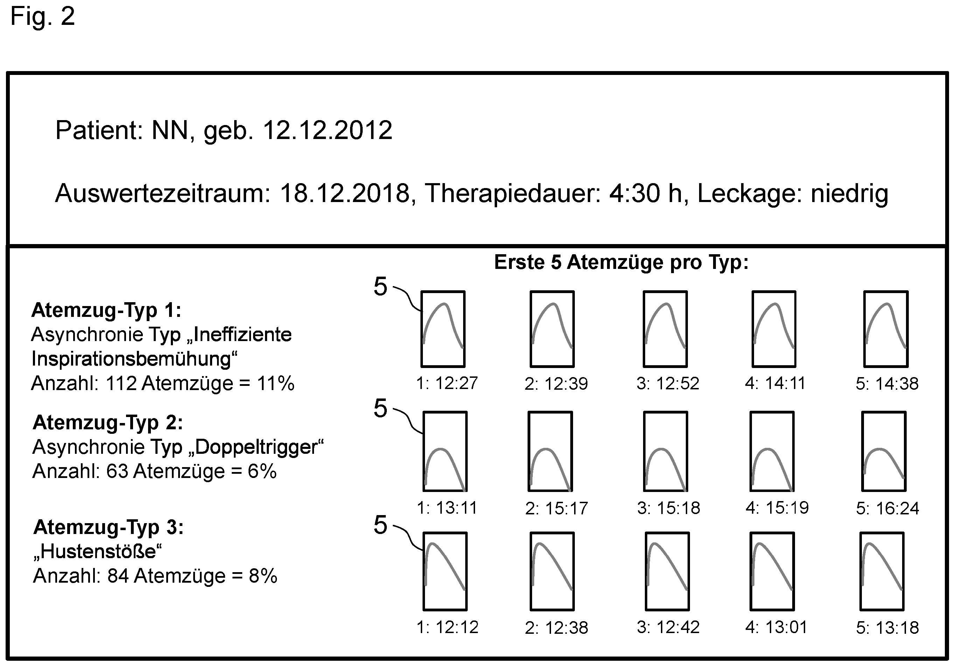

Fig. 2 stellt eine Übersichtsdarstellung von beispielhaft mittels des Atemzuganalysators 1 aus Fig. 1 gewonnenen Analyseergebnissen für eine verhältnismäßig kurze Gesamtanalysedauer dar, vorliegend 4,5 Stunden. Der Übersichtsdarstellung ist allgemein ein Zeitstempel, vorliegend ein Tagesdatum der durchgeführten Analyse sowie eine zusätzliche Leckageinformation (vorliegend "niedrig") während der Analysezeitdauer zu entnehmen. Des Weiteren weist die dargestellte Übersicht drei unterschiedliche Atemzugklassen, hier als Atemzug- bzw. Asynchronie-Typ 1 ("Ineffiziente Inspirationsbemühung"), Asynchronie-Typ 2 ("Doppelte Inspirationsbemühung/Doppeltrigger") und Atemzug-Typ 3 ("Hustenstöße") bezeichnet, aus, wobei für jede Atemzugklasse beispielhaft die während der Analysedauer erkannten fünf ersten (gespeicherten) Signalausschnitte 5 dargestellt sind. Jeder Signalausschnitt 5 ist zudem mit einem Zeitstempel, vorliegend die Uhrzeit der Bestimmung des jeweiligen Signalausschnitts 5, angegeben. Zu jeder der drei dargestellten Atemzugklassen ist ebenfalls die während der Analysezeitdauer insgesamt ermittelte absolute Häufigkeit (gespeichert im Häufigkeitszähler 8 des Atemzuganalysators 1 aus Fig. 1) der jeweiligen erkannten Atemzugklasse (vorliegend der Typen 1 bis 3) angegeben sowie die relative Häufigkeit in Prozent bezogen auf die während des Analysezeitraums insgesamt erkannten Atemzüge.

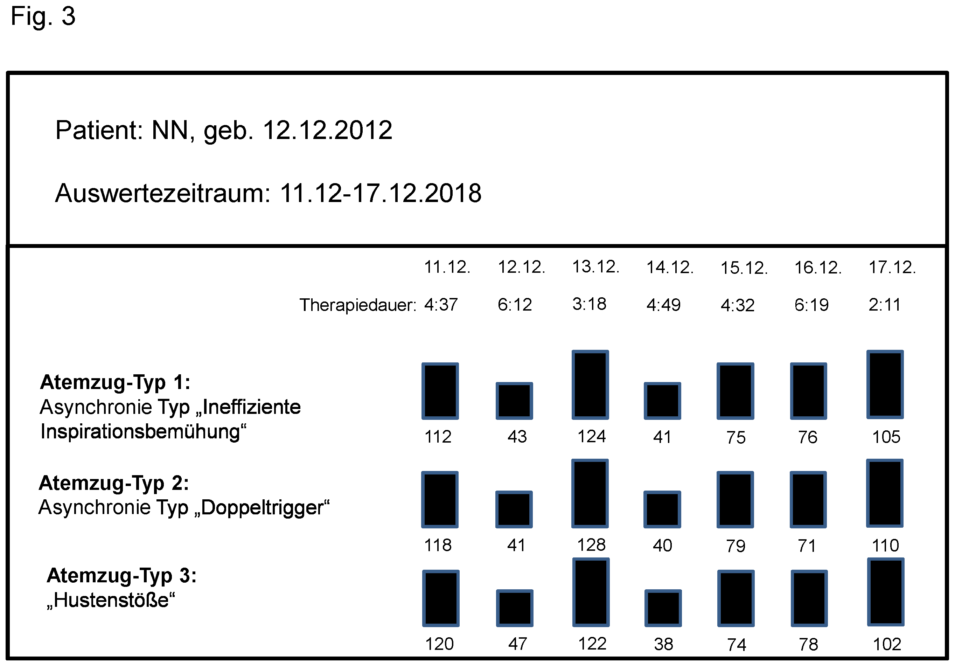

Fig. 3 stellt eine weitere Übersichtsdarstellung von mittels des Atemzuganalysators 1 aus Fig. 1 gewonnenen Analyseergebnissen für einen langen Analysezeitraum, vorliegend sieben Tage, dar, wobei an jedem Tag eine Analyse mit unterschiedlich langen Analysezeiträumen durchgeführt wurde, wie Fig. 3 anhand des jeweiligen Zeitstempels (Datum und Dauer) zu entnehmen ist. Die Analyseergebnisse sind jeweils für jede Atemzugklasse wie in Fig. 2 dargestellt, wobei in der Übersichtsdarstellung der Fig. 3 je Tag vorliegend die absoluten Häufigkeiten der erkannten Atemzugklassen während der jeweiligen Analysezeitdauer angegeben sind.

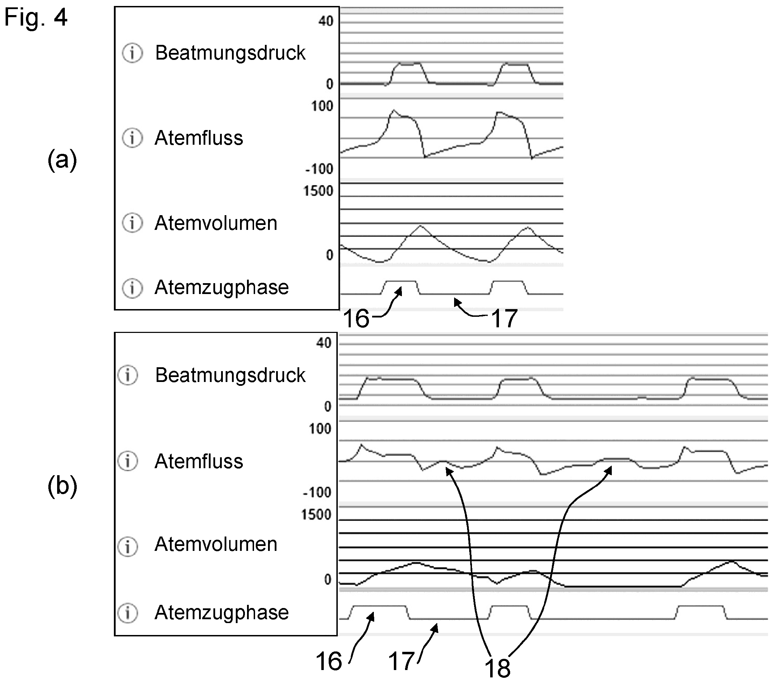

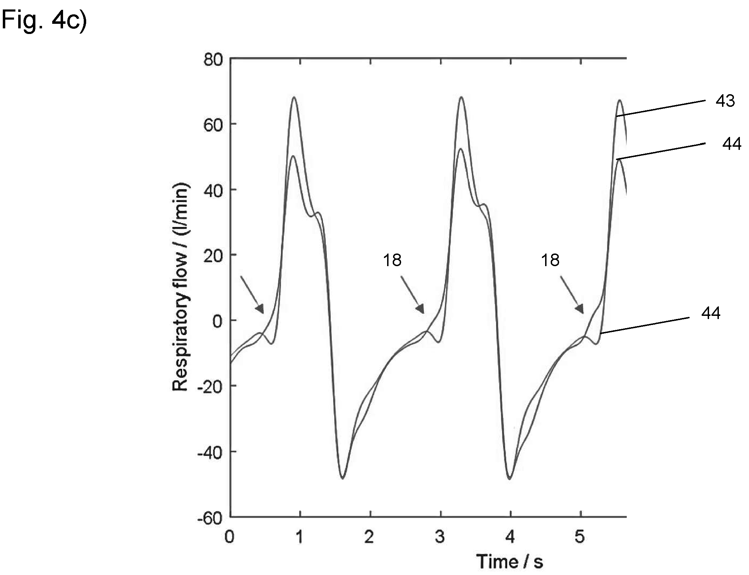

Die Fig. 4, 5, 6 und 7 stellen jeweils beispielhafte Signalverläufe eines einem Beatmungsdruck, einem Atemfluss und einem Atemvolumen entsprechenden Signals einer ersten, zweiten, dritten und vierten Atemzugklasse dar. In den jeweiligen Ansichten (a) sind die Signalverläufe für den Beatmungsdruck, Atemfluss, Atemvolumen und eine Atemzugphase (Inspirationsphase 16 und Exspirationsphase 17) eines unauffälligen Signalverlaufs dargestellt.

Fig. 4b stellt den für die in den Fig. 2 und 3 angegebene Atemzugklasse "Ineffiziente Inspirationsbemühung" (Typ 1), das heißt verpasste Atemzüge einer mit einem Beatmungsgerät beatmeten Person während der Exspirationsphase 17 des Beatmungsgeräts, typischen Signalverlauf an den Stellen 18 des Atemflusses dar. "Ineffiziente Inspirationsbemühungen" 18 sind ineffiziente Atemzugbemühungen des Patienten die einem Versuch der Inspiration entsprechen, jedoch meist unterschwellig sind und daher keine Umschaltung in die Phase der Inspiration (durch das Beatmungsgerät) auslösen.

"Ineffiziente Inspirationsbemühungen" 18 werden dadurch erkannt, dass der Atemfluss während der Exspirationsphase 17 analysiert wird. Üblicherweise würde der Atemfluss während der Exspirationsphase 17 dauerhaft unter die Nullline fallen, d.h. der Sensor registriert einen (negativen) Fluss, der in Richtung des Beatmungsgerätes gerichtet ist. "Ineffiziente Inspirationsbemühungen" weisen einen Verlauf auf, bei dem zunächst (für 0,1 bis 4 Sekunden oder für 10 - 90 % des Inspiratorischen Flusses) ein negativer Fluss aufgezeichnet wird. Dann wird eine Vorzeichenumkehr des Flusses erkannt, der Fluss nähert sich der Nulllinie an oder wird zeitweise positiv. Anschließend kann sich der Fluss wieder umkehren und erneut negativ werden und unter die Nulllinie fallen. Eine "Ineffiziente Inspirationsbemühung" kann generell dadurch erkannt werden, dass bei dem Atemfluss während der Exspirationsphase 17 zumindest zeitweise eine Vorzeichenumkehr des Flusses erkannt wird und/oder der Fluss zumindest zeitweise die Nullline wieder überschreitet.

Dies kann in einer Ausführungsform derart ermittelt werden, dass überprüft wird, ob der Ausatemfluss innerhalb eines ersten Zeitfensters, beispielsweise innerhalb einer festen Zeit oder eines Anteils der typischen Exspirationsdauer, zu Exspirationsbeginn eine bestimmte Schwelle (z.B. in l/min) überschreitet und danach innerhalb eines zweiten Zeitfensters abnimmt, bis er ein gewisse Schwelle (z.B. in l/min) unterschreitet oder sogar kurzzeitig ein positiver, also inspiratorischer Atemfluss entsteht oder zumindest die Ableitung des Atemflusses auf eine Abnahme der Schwelle des exspiratorischen Atemflusses hindeutet. Es wird dann überprüft, ob der Ausatemfluss innerhalb eines dritten Zeitfensters wieder zunimmt, also erneut ein stärkerer Ausatemfluss entsteht oder zumindest die Ableitung des Atemflusses auf eine erneute Zunahme der Schwelle des exspiratorischen Atemflusses hindeutet, noch bevor die nächste Inspiration erkannt wird.

Die Zeitfenster werden dabei bevorzugt in einem Bereich zwischen jeweils 0,1 und 3 Sekunden gewählt, bzw. jeweils zwischen 10 und 90% der typischen Ausatemdauer Alternativ kann die Ineffiziente Inspirationsbemühung auch durch eine Mustererkennung erkannt werden, die den Verlauf des Atemflusses analysiert und zuvor anhand von Testmustern angelernt wurde, Ineffiziente Inspirationsbemühungen zu erkennen.

Alternativ kann auch gemäß Fig. 4c ein idealer bzw. passiver Atemflussverlauf 43 ermittelt werden, der sich bei einer rein passiven Beatmung des Patienten, also ohne eigene Atemanstrengung, anhand des gemessenen Druckverlaufes ergeben würde, wenn man die Lunge des Patienten mit diesem Druckverlauf beaufschlagen würde. Dabei werden die Lungen-Parameter des Patienten wie Compliance und Resistance verschiedener Lungenareale zuvor ermittelt oder vom Beatmungsgerät geschätzt. Der passive Atemflussverlauf wird dann mit dem real gemessenen Atemflussverlauf 44 verglichen Weicht der real gemessene Atemflussverlauf vom ermittelten passiven Atemflussverlauf ab, so kann aus der Differenz unter anderem die Atemanstrengung des Patienten geschätzt werden. Wird innerhalb einer Exspiration eine vorübergehende Inspirationsbemühung 18, die eine vorher definierte Schwelle überschreitet, erkannt, so kann ebenfalls auf eine Ineffiziente Inspirationsbemühung geschlossen werden. Falls eine Inspirationsbemühung 18 erkannt wird, die erfolgreich einen Inspirationstrigger des Beatmungsgerätes auslöst, also eine effektive Inspirationsbemühung, so kann die Reaktionszeit des Einatemtriggers als zeitliche Differenz zwischen Inspirationsbemühung 18 und Anstieg des Therapiedruckes auf den inspiratorischen Therapiedruck ermittelt werden.

Fig. 4c den idealen bzw. passiven Atemflussverlauf 43 ohne Atemanstrengung und Atemwegsobstruktionen, der sich ergibt, wenn der Verlauf des Therapiedruckes auf ein Lungenmodell mit mindestens einer Compliance und Resistance beaufschlagt wird und den tatsächlichen Atemflussverlauf 44. Man erkennt die Inspirationsbemühungen 18 dadurch, dass der gemessene Atemfluss ansteigt, obwohl der Therapiedruck und damit auch der theoretische passive Atemfluss noch nicht ansteigen. Die zeitliche Differenz zwischen beiden Flusskurven wird als Trigger-Verzögerungszeit ermittelt. Ist die Trigger-Verzögerungszeit hoch, beispielsweise höher als 200 ms, so erhöht sich die Atemarbeit des Patienten und besteht die Gefahr des Auftretens von Ineffizienten Inspirationsbemühungen 18. Ist die Trigger-Verzögerungszeit sehr niedrig, beispielsweise niedriger als 50 ms, so besteht die Gefahr, dass Atemzüge auch ohne Inspirationsbemühung 18 des Patienten ausgelöst werden, sogenannte Selbsttriggerungen des Beatmungsgerätes. Beide Fälle, zu hohe als auch zu niedrige Trigger-Verzögerungszeit können den subjektiven und objektiven Erfolge der Beatmung beeinträchtigen.

Eine "Ineffiziente Inspirationsbemühung" sowie bevorzugt auch eine Aussage über die Trigger-Verzögerungszeit werden dem Arzt telemedizinisch übermittelt und/oder im Gerät angezeigt bzw. gespeichert. Ergänzend generiert das Gerät oder eine externe AnzeigeVorrichtung eine Therapieempfehlung oder führt diese automatisch durch. Hierbei wird bevorzugt die Triggerempfindlichkeit verändert oder die exspiratorische Zeitdauer verkürzt oder der inspiratorische Therapiedruck verändert, um durch eine Veränderung des Tidalvolumens den Atemantrieb bzw. die Spontan-Atemfrequenz zu verändern, so dass Inspirationsbemühungen 18 entweder früher (höherer Inspirationsdruck) oder später (niedrigerer Inspirationsdruck) auftreten. Alternativ kann auch der exspiratorische Therapiedruck bei verlängerter Trigger-Verzögerungszeit oder Ineffizienten Inspirationsbemühungen 18 erhöht werden. Dies ist insbesondere dann vorteilhaft, wenn der Patient durch in der Lunge eingeschlossene Luft einen erhöhten Lungendruck zum Ende der Exspiration aufweist (intrinsischer PEEP), der höher als der exspiratorische Therapiedruck des Beatmungsgerätes ist, so dass die Druckdifferenz iPEEP minus PEEP des Beatmungsgerätes durch den Patienten überwunden werden muss, um einen Trigger des Beatmungsgerätes auszulösen. Beispielsweise kann in einem solchen Fall der exspiratorische Therapiedruck zumindest zeitweise höher sein, als der inspiratorische Druck.

Bevorzugte Regeln für eine automatische Wahl eines sensitiveren Triggers, beispielsweisedurch Absenken der Flussschwelle zum Auslösen des Triggers, oder der Erhöhung des inspiratorischen Therapiedruckes zur Verlängerung der Ausatemphasen ohne erneuten inspiratorischen Atemantrieb oder Erhöhung des exspiratorischen Therapiedruckes zur Reduktion der Differenz zwischen iPEEP und PEEP des Beatmungsgerätes, die zum Auslösen eines Inspirationstriggers überwunden werden muss:
- Eine erkannte Ineffiziente Inspirationsbemühung
- Die Überschreitung einer definierten Anzahl von Ineffizienten Inspirationsbemühungen innerhalb eines Zeitfenster, beispielsweisemehr als 2 Ineffiziente Inspirationsbemühungen in 2 Minuten
- Eine erhöhte Trigger-Verzögerungszeit
- Eine erhöhte durchschnittliche Trigger-Verzögerungszeit innerhalb eines Zeitfensters, beispielsweise 2 min
- Die Überschreitung einer definierten Anzahl von Atemzügen mit erhöhter Trigger-Verzögerungszeit innerhalb eines Zeitfensters
- Die Summe aus Anzahl von Ineffizienten Inspirationsbemühungen und der Anzahl von Atemzügen mit erhöhter Trigger-Verzögerungszeit überschreitet eine definierte Anzahl innerhalb eines Zeitfensters
- Die Anzahl von Ineffizienten Inspirationsbemühungen pro Zeitfenster ist größer als die Anzahl von Atemzügen mit sehr kurzer Trigger-Verzögerungszeit innerhalb eines Zeitfensters
- Die Summe aus Anzahl von Ineffizienten Inspirationsbemühungen und der Anzahl von Atemzügen mit erhöhter Trigger-Verzögerungszeit ist größer als die Anzahl von Atemzügen mit sehr kurzer Trigger-Verzögerungszeit innerhalb eines Zeitfensters

Ein sensitiverer Trigger wird dann vorgegeben durch beispielsweise Absenken der Flussschwelle, die zum Auslösen des Triggers notwendig ist oder der Erhöhung des inspiratorischen Therapiedruckes zur Verlängerung der Ausatemphasen ohne erneuten inspiratorischen Atemantrieb oder Erhöhung des exspiratorischen Therapiedruckes zur Reduktion der Differenz zwischen iPEEP und PEEP des Beatmungsgerätes, die zum Auslösen eines Inspirationstriggers überwunden werden muss.

Für die Absenkung der Trigger-Sensitivität, die vorgegeben wird durch beispielsweise eine Anhebung der Flussschwelle zum Auslösen des Triggers oder einer Absenkung des inspiratorischen Therapiedruckes oder einer Absenkung des exspiratorischen Atemwegsdruckes, gelten die entsprechenden inversen Regeln.

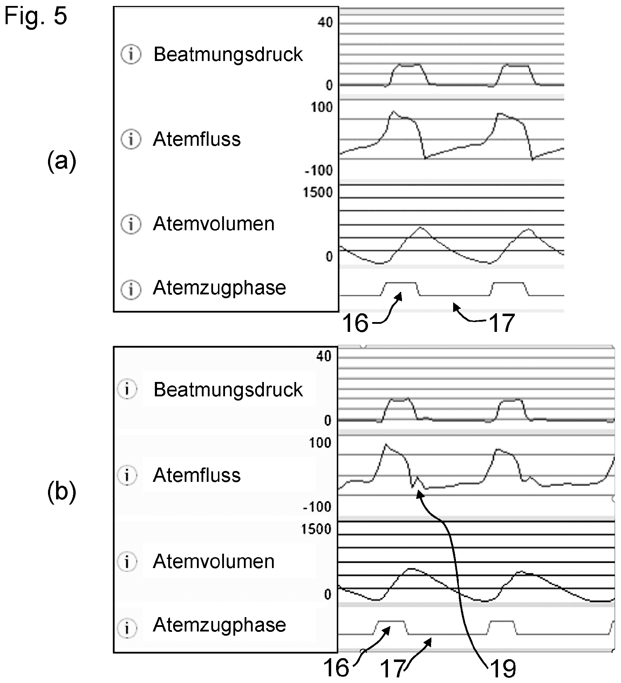

Fig. 5b stellt den für die in den Fig. 2 und 3 angegebene Atemzugklasse "Doppeltrigger" (Typ 2), das heißt weitere Inspirationsbemühung der mit einem Beatmungsgerät beatmeten Person zu Beginn der Exspirationsphase 17 des Beatmungsgeräts, typischen Signalverlauf an der Stelle 19 des Atemflusses dar.

Die Erkennung solcher Doppeltrigger erfolgt beispielsweise über eine stark verkürzte Ausatemzeit im Vergleich zur typischen, beispielsweise mittleren oder vorgegebenen Ausatemzeit, entweder einmalig oder bei x Atemzügen innerhalb eines Zeitfensters.

Die Erkennung solcher Doppeltrigger erfolgt beispielsweise auch über den gemessenen Fluss, der nach Umschaltung auf exspiratorischen Beatmungsdruck gegenüber dem theoretischen, passiven Atemfluss erhöht bleibt.

Bei Erkennung solcher Doppeltrigger erfolgt eine Meldung an den Betreuer über Telemonitoring oder am Display oder in Form gespeicherter Daten.

Bei Erkennung solcher Doppeltrigger erfolgt alternativ oder ergänzend eine automatische Reduktion der Sensitivität des Exspirationstriggers, also eine spätere Auslösung des Exspirationstriggers und/oder eine automatische Verlängerung einer vorgegebenen Inspirationszeit und/oder eine automatische Anwendung einer Trigger-Sperrzeit, innerhalb derer nach Umschaltung auf den exspiratorischen Beatmungsdruck kein erneuter Inspirationstrigger ausgelöst werden darf und/oder eine Erhöhung des inspiratorischen Beatmungsdruckes, damit sich die Lunge schneller mit Luft füllt und die Inspirationszeit des Patienten kürzer wird.

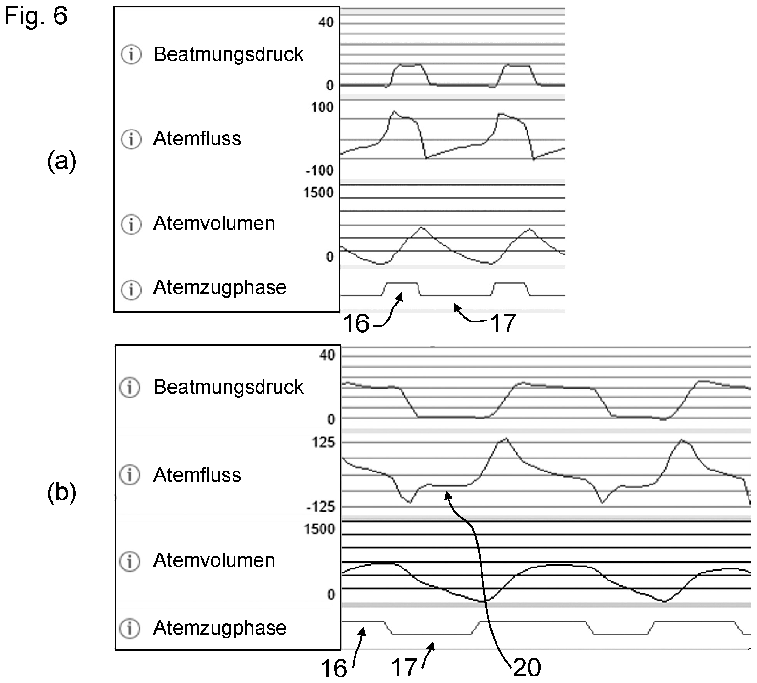

Fig. 6b stellt den für eine Atemzugklasse "Intrinsischer PEEP", das heißt während der Exspirationsphase 17 kann das von einer mit einem Beatmungsgerät beatmeten Person eingeatmete Luftvolumen nicht vollständig abgeatmet werden, typischen Signalverlauf an der Stelle 20 des Atemflusses dar.

Die Erkennung des Intrinsischen PEEP 20 erfolgt beispielsweise anhand von hinterlegten beispielhaften Kurvenverläufen von Fluss oder Volumen, die für einen Intrinsischen PEEP 20 repräsentativ sind. Aktuelle Kurvenverläufe werden dafür mit den hinterlegten Kurvenverläufen verglichen.

Die Erkennung des Intrinsischen PEEP 20 erfolgt alternativ auch über eine Auswertung der zeitlichen Fluss- bzw. Volumenverteilung innerhalb der Exspiration. Typisch für einen Intrinsischen PEEP 20 ist es, wenn am Anfang der Exspiration ein hoher Ausatemfluss erkannt wird, der anschließend abrupt abnimmt und im weiteren Verlauf der Exspiration in einen sehr niedrigen (beispielsweise niedriger als ein Schwellwert) Ausatemfluss übergeht. Die Erkennung des Intrinsischen PEEP 20 erfolgt alternativ durch Vergleich / Korrelation des Verlaufes des Ausatemflusses mit hinterlegten Vergleichskurven für Ausatemflüsse bei normaler Exspiration und bei Exspiration mit erhöhtem Widerstand, der zu einem iPEEP führen kann.

Die Erkennung des Intrinsischen PEEP 20 erfolgt auch durch Vergleich des realen Ausatemflusses mit dem theoretischen, passiven Ausatemfluss basierend auf dem Verlauf des Beatmungsdruckes und den vorab definierten oder zur Laufzeit geschätzten Lungenparametern des Patienten. Wenn der Ausatemfluss geringer ist, sich die Lunge also langsamer entleert, als dies bei einer idealen passiven Ausatmung der Fall wäre, muss ein zusätzlicher Widerstand in der Ausatmung den Atemfluss behindern. Es muss also eine Obstruktion der Atemwege vorliegen, eine sogenannte exspiratorische Flusslimitation, die häufig zu iPEEP führt.

Eine Erkennung einer Inspirationsbemühung 18 des Patienten und Auswertung des Ausatemflusses im Moment der Inspirationsbemühung 18 bzw. im Moment des Einsetzens der Hintergrundfrequenz des Beatmungsgerätes wird auch zur Erkennung des Intrinsischen PEEP 20 genutzt. Je größer der verbleibende exspiratorische Atemfluss im Moment des Auslösens der nächsten Inspiration, desto größer der intrinsische PEEP bzw. die in der Lunge eingeschlossene Luftmenge.

Erfindungsgemäß ist auch eine Zählung von Atemzügen mit einem hohen Grad an exspiratorischer Flusslimitation, in einem definierten Zeitraum (bzw. mit iPEEP-Verdacht) zwischen mehreren Minuten und einem Tag, vorgesehen, um einen Intrinsischen PEEP 20 zu erkennen.

Zudem kann die Bildung eines durchschnittlichen Grades an exspiratorischer Flusslimitation / an iPEEP, in einem definierten Zeitraum zwischen mehreren Minuten und einem Tag, erfolgen, um darüber einen Intrinsischen PEEP 20 zu erkennen.

Bei Erkennung eines Intrinsischen PEEP 20 erfolgt eine Meldung an den Betreuer über Telemonitoring und/oder am Display oder in gespeicherten Daten.

Insbesondere bei einer raschen Veränderung, also über Stunden bis wenige Tage, mit einer deutlichen Zunahme der exspiratorischen Flusslimitation, ist eine Warnung (Meldung) mit hoher Priorität vorteilhaft, da sich entweder die Qualität der Beatmung oder der Zustand des Patienten verschlechtert haben.

Bei Erkennung eines Intrinsischen PEEP 20 erfolgt alternativ eine automatische Anpassung der Druckrampe von IPAP zu EPAP, also des Übergangs vom inspiratorischen Beatmungsdruck zum exspiratorischen Beatmungsdruck. Wird diese Rampe flacher ausgeführt, oder in einem zweiten Teil der Exspiration flacher ausgeführt, oder wird der Druck in einem zweiten Teil der Exspiration wieder angehoben, so werden die unteren Atemwege am Kollaps gehindert, die exspiratorische Flusslimitation wird schwächer und damit auch der iPEEP.

Bei Erkennung eines Intrinsischen PEEP 20 erfolgt alternativ eine Anhebung des exspiratorischen Druckniveaus zur Schienung der unteren Atemwege und zur Reduktion der Atemarbeit zum Auslösen des inspiratorischen Triggers. Der ex. Druck kann dann zeitweise über dem ins. Druck liegen.

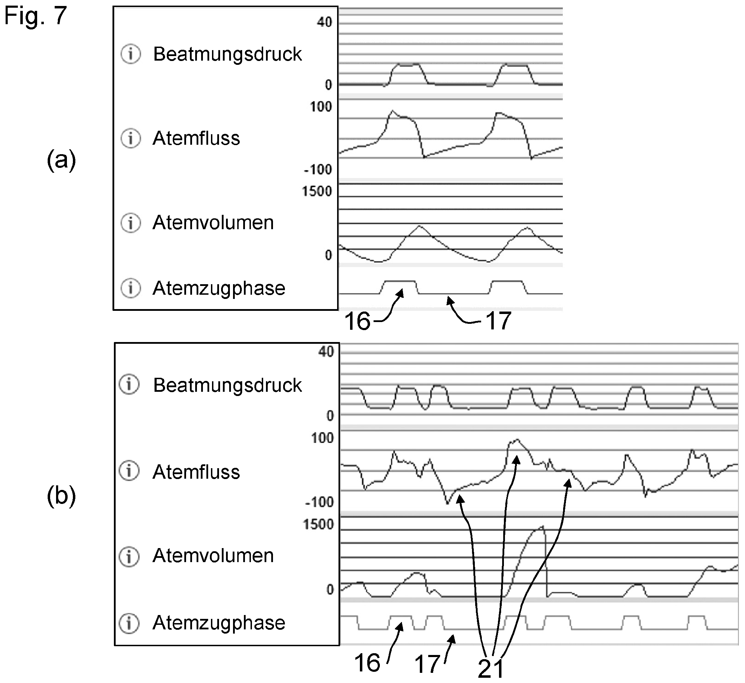

Fig. 7b stellt den für die in den Fig. 2 und 3 angegebene Atemzugklasse "Hustenstöße" (Typ 3), das heißt vergrößerte, forcierte und verlängerte Ein- und Ausatmung der mit einem Beatmungsgerät beatmeten Person, typischen Signalverlauf an der Stelle 21 des Atemflusses dar.

Die Erkennung von Hustenstößen 21 erfolgt beispielsweise über eine Mustererkennung des Exspirationsverlaufes anhand von Beispielkurven, die hinterlegt sind. Aktuelle Kurvenverläufe werden dafür mit den hinterlegten Kurvenverläufen verglichen.

Die Erkennung von Hustenstößen 21 erfolgt auch durch einen Vergleich / Korrelation des Verlaufes des Ausatemflusses, des Peak-Ausatemflusses oder -volumens mit den typischen, beispielsweise durchschnittlichen Ausatemflüssen, Peak-Ausatemflüssen oder -volumina des Patienten zur Erkennung einer forcierten Ausatmung, die auf einen Hustenstoß hindeutet. Ein Verdacht auf einen Hustenstoß besteht beispielsweise wenn der aktuelle Atemzug über 120% oder 150% oder 200% der typischen Atemzüge erreicht.

Die Erkennung von Hustenstößen 21 erfolgt alternativ durch eine zusätzliche Erkennung, ob einer vergrößerten Ausatmung auch eine vergrößerte Einatmung, gemessen am Volumen oder Flusses oder Peak-Flusses, vorausgeht, was den Verdacht auf Hustenstoß erhärtet. Die Erkennung von Hustenstößen 21 erfolgt ergänzend durch einen Vergleich des realen Ausatemflusses mit dem theoretischen, passiven Ausatemfluss basierend auf dem Verlauf des Beatmungsdruckes und den vorab definierten oder zur Laufzeit geschätzten Lungenparametern des Patienten. Wenn der Ausatemfluss größer ist, sich die Lunge also schneller entleert, als dies bei einer idealen passiven Ausatmung der Fall wäre, muss eine forcierte Ausatmung vorliegen. Die Schwelle der forcierten Ausatmung kann beispielsweise über die Volumendifferenz zwischen der realen und der idealen passiven Ausatmung ermittelt werden oder aus der Differenz der beiden Flusskurven. Übersteigt die Differenz des Volumens oder des Flusses einen gewissen Wert, so wird ein Hustenstoß erkannt. Die Erkennung von Hustenstößen 21 erfolgt auch durch einen Vergleich der Ableitung des Atemflusses nach einem der genannten Kriterien.

Bei Erkennung von Hustenstößen 21 erfolgt eine Meldung an den Betreuer über Telemonitoring und/oder am Display oder in gespeicherten Daten. Insbesondere bei einer raschen Veränderung, also über Stunden bis wenige Tage, mit einer deutlichen Zunahme der exspiratorischen Flusslimitation, ist eine Warnung (Meldung) mit hoher Priorität vorteilhaft, da sich entweder die Qualität der Beatmung oder der Zustand des Patienten verschlechtert haben.

Insbesondere bei einer raschen Veränderung, also über Stunden bis wenige Tage, mit einer deutlichen Zunahme der Husten-Häufigkeit, ist eine Warnung mit hoher Priorität vorteilhaft, da sich entweder die Qualität der Beatmung oder der Zustand des Patienten verschlechtert haben.

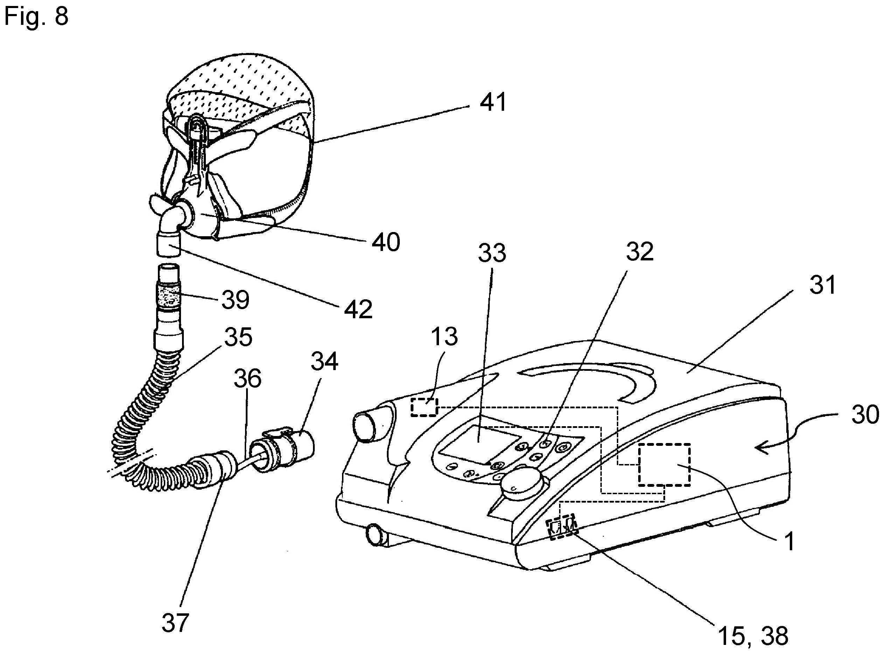

Fig. 8 stellt eine perspektivische Ansicht eines Ausführungsbeispiels eines Beatmungsgeräts 30 zur Beatmung einer Person mit einem Atemgas aus einer Atemgasquelle (nicht dargestellt), die in einem Innenraum eines Gerätegehäuses 31 angeordnet sein kann, gemäß der Erfindung dar. Im Bereich des Gerätegehäuses 31 sind ein Bedienelement 32 und eine Anzeigeeinrichtung 33 vorgesehen, wobei die Anzeigeeinrichtung 33 auch als kombinierte Bedien- und Anzeigeeinrichtung beispielsweise mit einer berührungsempfindlichen Eingabeeinheit ausgebildet sein kann. Über eine Kopplung 34 ist ein Verbindungsschlauch 35 angeschlossen. Entlang des Verbindungsschlauches 35 kann ein zusätzlicher Druckmessschlauch 36 verlaufen, der über einen Druckeingangsstutzen 37 mit dem Gerätegehäuse 31 verbindbar ist. Zur Ermöglichung einer Datenübertragung weist das Beatmungsgerät 30 zumindest eine oder auch eine Vielzahl von Datenübertragungsschnittstelle(n) 15, 38 auf.

Bei dem in Fig. 8 dargestellten Beatmungsgerät 30 ist der in Fig. 1 gezeigte erfindungsgemäße Atemzuganalysator 1 als Teil des Beatmungsgerätes 30 ausgeführt. Wie Fig. 8 weiter zu entnehmen ist, ist der Atemzuganalysator 1 mit der in Fig. 1 dargestellten, vorliegend dem Beatmungsgerät 30 zugeordneten Sensoreinheit 13 sowie mit den Datenübertragungsschnittstellen 15 bzw. 38 gekoppelt.

Ein an sich bekannter Anfeuchter (nicht dargestellt) kann zudem adaptiert werden. Im Bereich eines dem Gerätegehäuse 31 abgewandten Endes des Verbindungsschlauchs 35 ist ein Ausatmungselement 39 angeordnet. Ebenfalls kann ein Ausatemventil (nicht explizit dargestellt) verwendet werden.

Fig. 8 ist weiter ein als Beatmungsmaske 40 ausgebildetes Patienten-Interface zu entnehmen, das vorliegend als Nasalmaske realisiert ist. Eine Fixierung im Bereich eines Kopfes einer zu beatmenden Person kann über eine Kopfhaube 41 erfolgen. Im Bereich seines dem Verbindungsschlauch 35 zugewandten Endes weist das Patienten-Interface 40 ein Kupplungselement 42 auf.

Über die Schnittstelle 38 kann beispielsweise eine Eingabe und/oder Ausgabe von Daten, wie beispielsweise Totraumvolumen, erfolgen. Die Datenübertragungsschnittstelle(n) 15, 38 können kabelgebunden, als Infrarot-Schnittstelle, als Bluetooth-Schnittstelle oder USB realisiert sein. Bevorzugt ist auch ein Speicherkartenschacht (nicht dargestellt) vorgesehen. Die Schnittstelle 15, 38 kann auch als LAN-Schnittstelle oder als sonstige Datenübertragungsschnittstelle zur Anbindung an ein Netzwerk, das Internet etc. ausgeführt sein. Im Bereich des Gerätegehäuses 31 kann ein Sauerstoffzuschaltventil (nicht dargestellt) an das Beatmungsgerät 30 adaptiert sein. Es ist denkbar, das Atemgas zusätzlich mit Sauerstoff anzureichern, um die Patientenversorgung zu verbessern.

Über die Schnittstellen 38 - beispielsweise als Speicherkartenschacht oder USB ausgeführt - können auch therapiefremde Daten in das erfindungsgemäße Beatmungsgerät 30 geladen werden beziehungsweise von diesem zu einem geräteexternen Datenempfänger geladen werden. Der Anwender muss - werden vom Beatmungsgerät 30 externe Speichermedien erkannt - eine Abfrage auf der Anzeigeeinrichtung 33 über das Bedienfeld 32 bestätigen, woraufhin die Daten wahlweise im Bereich des Beatmungsgerätes 30 gespeichert oder ausgeführt werden.

Über die Schnittstelle 38 kann die Eingabe und/oder Ausgabe von telemedizinischen Daten erfolgen. Dazu werden über die Datenübertragungsschnittstelle 38 beispielsweise Mobilfunk- oder Nahfeldfunkdaten empfangen/gesendet oder WLAN oder Bluetooth oder Netzwerkdaten.

Das Beatmungsgerät 30 ist so ausgelegt, dass es über den Schlauch 35 und das Patienten-Interface 40 mit der zu beatmenden Person bzw. dem Patienten verbunden werden kann, um eine Beatmung durch das Gerät 30 bereitzustellen. Die Atemgasquelle kann beispielsweise als ein Elektromotor mit einem von diesem angetriebenen Gebläserad (beides nicht dargestellt) ausgebildet sein. Die Sensoreinheit 13 ist zur Ermittlung des Beatmungsdrucks und/oder des Atemflusses und/oder des Atemvolumens des Atemgases ausgebildet. Das Beatmungsgerät 30 weist eine nicht dargestellte elektronische Steuereinheit auf, die so ausgebildet ist, dass für jeden Atemzug auf der Basis eines vorbestimmten Wertes für den Patienten und/oder auf Basis von Messsignalen der Sensoreinheit 13 ein Atemgasdruck bestimmt wird und die Atemgasquelle derart reguliert wird, dass der gewünschte Atemgas- bzw. Beatmungsdruck erzeugt wird.

Ein besonders bevorzugter Einsatzschwerpunkt des hierin offenbarten Atemzuganalysators bzw. des Atemzuganalyseverfahrens ist die Überwachung von Exspirationsphasen der zu beatmenden Person, zum Beispiel während des Schlafs. Mehrere Komplikationszustände bei einer Beatmung lassen sich durch den Atemflussverlauf während der Exspiration erkennen. Durch eine frühzeitige Reaktion (beispielsweise durch Medikation oder Optimierung der Beatmungseinstellungen) lässt sich das Ergebnis der Beatmung deutlich verbessern.

So kann der exspiratorische Atemflussverlauf der beatmeten Person beispielsweise bereits im Beatmungsgerät 30 ausgewertet werden, sofern dieses eine entsprechende Auswerteeinheit aufweist, wobei bevorzugt mindestens 2 von 4 Komplikationszuständen erkannt werden: Schweregrad einer exspiratorischen Flusslimitation, Schweregrad eines intrinsischen PEEP, Häufigkeit von verpassten Atemzügen (ausbleibender Inspirationstrigger bzw. Ineffiziente Inspirationsbemühung), Häufigkeit von Hustenstößen. Die Erkennung der jeweiligen Atemzugklassen aus den bestimmten Signalausschnitten kann zum Beispiel durch Mustererkennung, Vergleich des Exspirationsverlaufes mit hinterlegten Referenzverläufen, Auswertung des Atemflusses und/oder der Steigung des Atemflusses bzw. des ausgeatmeten Volumens zu bestimmten Zeitpunkten im Verlauf der Exspiration realisiert werden. Eine Speicherung und Verarbeitung der erkannten Komplikationen kann im Beatmungsgerät selbst erfolgen und/oder über die Datenübertragungsschnittstelle 38 an einen Datenempfänger übertragen werden. Vorzugsweise erfolgt eine Übermittlung von erkannten Komplikationszuständen an eine Überwachungseinheit, beispielsweise in einem Krankenhaus oder einem Pflegeheim, an einen Monitor und bei einer häuslichen Beatmung an einen Telemonitoring-Server. Außerdem kann eine visuelle Darstellung/akustische Erzeugung eines Hinweises oder eines Alarmes an der Überwachungseinheit ausgelöst werden, falls die Häufigkeit oder der Schweregrad der Komplikationen einen festgelegten Grenzwert überschreiten.

Um die vorgenannten Atmungsereignisse zu erkennen, kann jeweils der Atemfluss während der Exspirationsphase 17 des Beatmungsgeräts 30 ausgewertet werden, optional in Kombination mit dem Beatmungsdruck oder der von der Sensoreinheit 14 (Fig. 1) erfassten Drehzahl eines Beatmungsgebläses der Atemgasquelle und/oder der erfassten Leckageverluste.

Für jedes der Muster oder jedes der vorgenannten Atmungsereignisse, die in der Exspiration erkennbar sind, gibt es erfindungsgemäß verschiedene Möglichkeiten zur Erkennung. Diese können einzeln oder kombiniert angewandt werden, und sind auch nur als Ausführungsbeispiele zu sehen. Auch muss nicht nach allen der beschriebenen Muster gesucht werden, mindestens jedoch nach einem.

Die Auswertung, beispielsweise durch eine innerhalb des Beatmungsgeräts 30 vorgesehene oder alternativ/zusätzlich durch eine geräteexterne Auswerteeinheit (beides nicht dargestellt), kann einen oder eine Kombination der folgenden Verfahrensschritte beinhalten:
a) Auswertung der exspiratorischen Flussamplituden, insbesondere Maxima und Minima und deren zeitliches Auftreten:
   - Maximum während des ersten Teils der Exspirationsphase bedeutet: zu kurze Inspirationszeit, weitere Inspirationsbemühungen
   - Maximum während des weiteren Verlaufs der Exspirationsphase bedeutet: verpasster Atemzug
   - Minimum tiefer als in vorangegangen Atemzügen bedeutet: Hustenstoß
   - Atemfluss zu Inspirationsbeginn noch negativ bedeutet: intrinsischer PEEP
b) Auswertung des exspiratorischen Volumens und ggf. eines während der Exspirationsphase messbaren inspiratorischen Volumens:
   - Positives Volumen in einem Abschnitt der Exspirationsphase bedeutet: verpasster Atemzug
   - Exspiratorisches Volumen ist vergrößert im Vergleich zu vorangegangenen Exspirationen bedeutet: Hustenstoß
   - Exspiratorisches Volumen kleiner als inspiratorisches Volumen bedeutet: intrinsischer PEEP
c) Auswertung der Ableitung (= Steigung) des Atemflusses:
   - Anstieg gefolgt von Abfall während des ersten Teils der Exspirationsphase bedeutet: zu kurze Inspirationszeit, weitere Inspirationsbemühungen
   - Anstieg gefolgt von Abfall während des weiteren Verlaufs der Exspirationsphase bedeutet: verpasster Atemzug
   - Abfall steiler als in vorangegangen Atemzügen bedeutet: Hustenstoß
d) Auswertung der Volumenverteilung der Ausatmung während der Exspirationsphase:
   - Volumenanteile vor allem am Anfang und am Ende eines Atemzugs bedeutet: verpasster Atemzug
   - Volumenanteile stark am Anfang eines Atemzugs gebündelt bedeutet: intrinsischer PEEP
e) Auswertung der Kontur des Atemflusses und/oder des Volumenverlaufs:
   - Korrelation oder anderes Ähnlichkeitsmaß mit hinterlegten oder gelernten Referenzkonturverläufen
   - Mustererkennung, beispielsweise mit neuronalen Netzen oder Support Vector Machines oder Principal Component Analysis oder Fuzzy-Logik

Statt der hierin beschriebenen Auswertung des Atemflusses über der Zeit kann alternativ auch der Atemfluss und/oder das Atemvolumen über dem Beatmungsdruck (P/V-Diagramm) ausgewertet werden.

Der hierin offenbarte erfindungsgemäße Atemzuganalysator, das Beatmungsgerät sowie das erfindungsgemäße Verfahren zur Atemzuganalyse sind nicht auf die hierin jeweils offenbarten Ausführungsformen beschränkt, sondern umfassen auch gleich wirkende weitere Ausführungsformen, die sich aus technisch sinnvollen weiteren Kombinationen der hierin beschriebenen Merkmale des Atemzuganalysators, des Beatmungsgeräts sowie des Atemzuganalyseverfahrens ergeben. Insbesondere sind die hierin vorstehend in der allgemeinen Beschreibung und der Figurenbeschreibung genannten und/oder in den Figuren alleine gezeigten Merkmale und Merkmalskombinationen nicht nur in den jeweils hierin explizit angegebenen Kombinationen, sondern auch in anderen Kombinationen oder in Alleinstellung verwendbar, ohne den Rahmen der vorliegenden Erfindung zu verlassen.

In bevorzugter Ausführung wird der erfindungsgemäße Atemzuganalysator in einem Beatmungsgerät verwendet, um unterschiedliche Atmungsereignisse einer von dem Beatmungsgerät mit einem Atemgas beatmeten Person, beispielsweise während des Schlafs, automatisch zu erkennen und für eine nachgeordnete Verarbeitung, wie hierin beschrieben, bereitzustellen.

## Patentansprüche

1. Atemzuganalysator zur Erkennung von Atmungsereignissen einer mit einem Atemgas beatmeten Person, aufweisend eine elektronische Rechen- und Speichereinheit (2), die ausgebildet und eingerichtet ist, ein einem Beatmungsdruck und/oder einem Atemfluss und/oder einem Atemvolumen des der Person zugeführten Atemgases entsprechendes Signal (3) zu empfangen und während einer vorbestimmten Analysezeitdauer mittels eines Verlaufsanalysators (4) einen Verlauf des Signals (3) zu erfassen, **dadurch gekennzeichnet, dass** die Rechen- und Speichereinheit (2) ausgebildet und eingerichtet ist, aus dem Signalverlauf einen Signalausschnitt (5) zu bestimmen, der anhand einer vordefinierten oder einer aus dem Signalverlauf erkannten Ausschnittzeitdauer (6) kürzer als die Analysezeitdauer festgelegt ist, und den bestimmten Signalausschnitt (5) mittels eines Komparators (7) einer von mehreren Atemzugklassen zuzuordnen und jeweils eine Auftrittshäufigkeit der während der Analysezeitdauer zugeordneten Atemzugklasse in einem für jede Atemzugklasse vorgesehenen Häufigkeitszähler (8) in der Speichereinheit (2) zu speichern sowie wenigstens einen, jedoch weniger als die im Häufigkeitszähler (8) erfasste Auftrittshäufigkeit, der während der Analysezeitdauer bestimmten Signalausschnitte (5) für jede zugeordnete Atemzugklasse in der Speichereinheit (2, 9) zu speichern.

2. Atemzuganalysator nach Anspruch 1, **dadurch gekennzeichnet, dass** die Rechen- und Speichereinheit (2) ausgebildet und eingerichtet ist, je Atemzugklasse lediglich eine vorbestimmte maximale Anzahl an Signalausschnitten (5) in der Speichereinheit (2, 9) zu speichern.

3. Atemzuganalysator nach einem der vorhergehenden Ansprüche, **dadurch gekennzeichnet, dass** die Ausschnittzeitdauer (6) des Signalausschnitts (5) anhand wenigstens eines vorbestimmten Signalverlaufskriteriums festlegbar ist, das ausgewählt ist aus der Gruppe aufweisend eine Steigung und/oder einen Steigungsverlauf des Signalverlaufs, ein Auftreten eines Maximums und/oder Minimums des Signalverlaufs und dergleichen.

4. Atemzuganalysator nach einem der vorhergehenden Ansprüche, **dadurch gekennzeichnet, dass** die Ausschnittzeitdauer (6) derart bemessen ist, dass der Signalausschnitt (5) wenigstens einen vollständigen Atemzug beinhaltet, vorzugsweise 1 bis 3 Atemzüge oder wenigstens 3 bis maximal 10 Atemzüge und dass der gespeicherte Signalausschnitt (5) mit einem Zeitstempel seiner Bestimmung versehen ist.

5. Atemzuganalysator nach einem der vorhergehenden Ansprüche, **dadurch gekennzeichnet, dass** der Komparator (7) ausgebildet und eingerichtet ist, den Signalausschnitt (5) der Atemzugklasse mittels Mustererkennung und/oder mittels eines Vergleichs des Signalverlaufs im Signalausschnitt (5) mit einer vorbestimmten Anzahl an vorab in der Speichereinheit (2) gespeicherten, vordefinierten Referenzsignalverläufen (10) und/oder mittels einer erfassten Steigung und/oder eines Steigungsverlaufs des Signalverlaufs im Signalausschnitt (5) und/oder mittels eines oder mehrerer erfasster Maxima und/oder Minima des Signalverlaufs im Signalausschnitt (5) zuzuordnen und/oder dass der Komparator (7) ausgebildet und eingerichtet ist, eine vorbestimmte Anzahl an ausgewählten, den Atemzugklassen während der Analysezeitdauer zugeordneten Signalausschnitten (5) als neu hinzugefügte, adaptiv gelernte Referenzsignalverläufe (11) in der Speichereinheit (2) zu speichern und bei der Zuordnung zukünftig erfasster Signalausschnitte (5) zu den Atemzugklassen zu berücksichtigen.

6. Atemzuganalysator nach einem der vorhergehenden Ansprüche, **dadurch gekennzeichnet, dass** die Atemzugklassen die Atemzugstypen Inspiration, Exspiration, Pause und Husten beinhalten, vorzugsweise zusätzlich auch Atemzugtypen, die eine inspiratorische Flusslimitation und/oder eine exspiratorische Flusslimitation und/oder einen intrinsischen PEEP 20 repräsentieren und/oder Atemzug-Asynchronietypen wie eine ineffiziente Atemzugbemühung 18 und/oder eine doppelte Atemzugbemühung (Doppeltrigger) 19.

7. Atemzuganalysator nach einem der vorhergehenden Ansprüche, **dadurch gekennzeichnet, dass** die Atemzugklasse ineffiziente Atemzugbemühung 18 dadurch erkannt wird, dass der Atemfluss während der Exspirationsphase 17 analysiert wird und dass der Atemfluss während der Exspirationsphase 17 zumindest zeitweise eine Vorzeichenumkehr aufweist und/oder dass die Atemzugklasse ineffiziente Atemzugbemühung 18 dadurch erkannt wird, dass die Ineffiziente Inspirationsbemühung 18 durch eine Mustererkennung erkannt wird, die den Verlauf des Atemflusses vergleicht mit zuvor gespeicherten Testmustern, die Ineffiziente Inspirationsbemühungen repräsentieren.

8. Atemzuganalysator nach einem der vorhergehenden Ansprüche, **dadurch gekennzeichnet, dass** die Atemzugklasse ineffiziente Atemzugbemühung 18 dadurch erkannt wird, dass analysiert wird, ob der Ausatemfluss innerhalb eines ersten Zeitfensters zu Exspirationsbeginn eine bestimmte Schwelle (z.B. in l/min) überschreitet und danach innerhalb eines zweiten Zeitfensters abnimmt, bis er eine gewisse Schwelle unterschreitet oder sogar kurzzeitig ein positiver, also inspiratorischer Atemfluss entsteht oder zumindest die Ableitung des Atemflusses auf eine Abnahme des exspiratorischen Atemflusses hindeutet, wobei dann überprüft wird, ob der Ausatemfluss innerhalb eines dritten Zeitfensters wieder zunimmt, also erneut ein stärkerer Ausatemfluss entsteht oder zumindest die Ableitung des Atemflusses auf eine erneute Zunahme des exspiratorischen Atemflusses hindeutet, noch bevor die nächste Inspiration erkannt wird.

9. Atemzuganalysator nach einem der vorhergehenden Ansprüche, **dadurch gekennzeichnet, dass** die Atemzugklasse Doppeltrigger 19 erkannt wird durch einen Vergleich der Ausatemzeit mit einer Vielzahl von vorausgehenden (typischen) Ausatemzeiten oder einer durchschnittlichen Ausatemzeit oder einer vorgegebenen Ausatemzeit und/oder dass die Atemzugklasse Doppeltrigger 19 erkannt wird durch einen Vergleich des gemessenen Flusses, mit einem theoretischen Atemfluss, wobei der gemessene Fluss der nach Umschaltung auf exspiratorischen Beatmungsdruck gegenüber dem theoretischen Atemfluss erhöht bleibt.

10. Atemzuganalysator nach einem der vorhergehenden Ansprüche, **dadurch gekennzeichnet, dass** die Erkennung der Atemzugklasse des Intrinsischen PEEP 20 anhand von hinterlegten beispielhaften Kurvenverläufen von Fluss oder Volumen, die für einen Intrinsischen PEEP 20 repräsentativ sind, erfolgt, wobei aktuelle Kurvenverläufe dafür mit den hinterlegten Kurvenverläufen verglichen werden und/oder dass die Erkennung der Atemzugklasse des Intrinsischen PEEP 20 über eine Auswertung der zeitlichen Fluss- oder Volumenverteilung innerhalb der Exspiration erfolgt, wobei am Anfang der Exspiration ein hoher Ausatemfluss erkannt wird, der anschließend abnimmt und im weiteren Verlauf der Exspiration in einen niedrigen Ausatemfluss übergeht.

11. Atemzuganalysator nach einem der vorhergehenden Ansprüche, **dadurch gekennzeichnet, dass** die Erkennung der Atemzugklasse des Intrinsischen PEEP 20 über eine Erkennung einer Inspirationsbemühung 18 des Patienten und eine Auswertung des Ausatemflusses im Zeitbereich der Inspirationsbemühung 18 erfolgt, wobei der verbleibende exspiratorische Atemfluss im Zeitbereich des Auslösens der nächsten Inspirationsphase, repräsentativ für das Ausmaß des intrinsischen PEEP ist.

12. Atemzuganalysator nach einem der vorhergehenden Ansprüche, **dadurch gekennzeichnet, dass** die Erkennung der Atemzugklasse Hustenstoß 21 über eine Mustererkennung des Exspirationsverlaufes von Fluss oder Volumen anhand von hinterlegten Verläufen erfolgt, die repräsentativ für Hustenstöße sind, wobei aktuelle Verläufe dafür mit den hinterlegten Verläufen verglichen werden.

13. Atemzuganalysator nach einem der vorhergehenden Ansprüche, **dadurch gekennzeichnet, dass** die Erkennung der Atemzugklasse Hustenstoß 21 über einen Vergleich des Verlaufes des aktuellen Ausatemflusses, des Peak-Ausatemflusses oder -volumens mit den typischen vorhergehenden Ausatemflüssen, Peak-Ausatemflüssen oder -volumina des Patienten zur Erkennung einer forcierten Ausatmung erfolgt, die auf einen Hustenstoß hindeutet, wobei ein Hustenstoß erkannt wird, wenn der aktuelle Atemzug über 70% der typischen Atemzüge erreicht und/oder dass der Komparator (7) ausgebildet und eingerichtet ist, Zusatzinformationen über den Atemzugtyp wie spontaner oder mandatorischer Atemzug und/oder über eine Drehzahl eines der Person das Atemgas zuführenden Beatmungsgebläses und/oder über einen Leckageverlust des der Person zugeführten Atemgases zu empfangen und bei der Zuordnung des Signalausschnitts (5) zu den Atemzugklassen zu berücksichtigen.

14. Beatmungsgerät zur Beatmung einer Person mit einem Atemgas, aufweisend eine Sensoreinheit (13) zur Bestimmung eines Beatmungsdrucks und/oder eines Atemflusses und/oder eines Atemvolumens des der Person zugeführten Atemgases, **gekennzeichnet durch** einen Atemzuganalysator (1) nach einem der vorhergehenden Ansprüche, wobei die Sensoreinheit (13) mit der Rechen- und Speichereinheit (2) des Atemzuganalysators (1) in datenübertragender Weise gekoppelt ist und eingerichtet ist, ein dem bestimmten Beatmungsdruck und/oder dem bestimmten Atemfluss und/oder dem bestimmten Atemvolumen des Atemgases entsprechendes Signal (3) zu erzeugen und der Rechen- und Speichereinheit (2) zuzuführen.

15. Verfahren zur Erkennung von Atmungsereignissen einer mit einem Atemgas beatmeten Person, bei dem mittels einer Sensoreinheit (13) ein einem Beatmungsdruck und/oder einem Atemfluss und/oder einem Atemvolumen des der Person zugeführten Atemgases entsprechendes Signal (3) erzeugt wird und während einer vorbestimmten Analysezeitdauer ein zeitlicher Verlauf des Signals (3) ermittelt wird, **dadurch gekennzeichnet, dass** aus dem Signalverlauf ein Signalausschnitt (5) bestimmt wird, der anhand einer vordefinierten oder einer aus dem Signalverlauf erkannten Ausschnittzeitdauer (6) kürzer als die Analysezeitdauer festgelegt wird, und der bestimmte Signalausschnitt (5) einer von mehreren Atemzugklassen zugeordnet wird, wobei jeweils eine Auftrittshäufigkeit der während der Analysezeitdauer zugeordneten Atemzugklasse in einem für jede Atemzugklasse vorgesehenen Häufigkeitszähler (8) gespeichert wird sowie wenigstens einer, jedoch weniger als die im Häufigkeitszähler (8) erfasste Auftrittshäufigkeit, der während der Analysezeitdauer bestimmten Signalausschnitte (5) für jede zugeordnete Atemzugklasse gespeichert wird.
